# EUROPEAN PATENT SPECIFICATION

(11) **EP 4 610 367 B1**
(45) Date of publication and mention of the grant of the patent: **04.02.2026**
(21) Application number: 24160876.9
(22) Date of filing: 01.03.2024
(51) Int. Cl.: C12Q 1/6806, C12Q 1/6853

(54) **METHODS FOR ACCURATE PARALLEL AMPLIFICATION, DETECTION AND QUANTIFICATION OF NUCLEIC ACIDS**
VERFAHREN ZUR GENAUEN PARALLELEN AMPLIFIKATION, DETEKTION UND QUANTIFIZIERUNG VON NUKLEINSÄUREN
PROCÉDÉS D'AMPLIFICATION, DE DÉTECTION ET DE QUANTIFICATION PARALLÈLES PRÉCISES D'ACIDES NUCLÉIQUES

(43) Date of publication of application: 03.09.2025
(73) Proprietor: Genomill Health Oy, 20520 Turku (FI)
(72) Inventor: Pursiheimo, Juha-Pekka, 21530 Paimio (FI); Hirvonen, Tatu, 20540 Turku (FI); Musku, Anna, 21800 Kyrö (FI); Tamminen, Manu, 20500 Turku (FI); Rantasalo, Tuula, 21270 Nousiainen (FI)
(74) Representative: Moosedog Oy

(56) References cited:
- EP-A1- 4 332 238
- WO-A1-2019/038372
- WO-A1-2023/139309

## Description

### TECHNICAL FIELD

The present invention disclosure relates to improved next generation DNA sequencing methods for accurate and massively parallel amplification and detection and quantification of one or more nucleic acid targets. More particularly, the disclosure is related to methods using probes for detecting and quantifying genetic targets in complex DNA pools primarily used for genetic target and variant detection. The invention uses one or more target-specific nucleic acid probes per genetic target, a loop oligo and one or more bridge oligos.

### BACKGROUND

With the advancement in the technology to study genetic variation, detection of the same in plants and animals is not cumbersome. However, detecting and accurately quantifying genetic variations such as mutations, in particular in samples having weak signals is currently still cumbersome, laborious and expensive, despite decreased sequencing costs. Various problems can be expressed more accurately such as specificity in order to detect genetic signals against a consensus background, sensitivity in order to detect weak genetic signals, accuracy for accurate quantification of the detected signals, throughput number of targeted genetic targets per assay, cost per assay, scaling to determine the assay cost scale when assaying multiple samples in parallel and turn-over to determine how long is the time from sampling to the results.

Currently, the typical quantification methods for liquid biopsies and conceptually similar assays (such as antibiotic resistance gene detection) include quantitative PCR (qPCR), array qPCR, digital PCR, Multiplex Ligation-dependent Probe Amplification (MLPA) or quantification from next-generation DNA sequencing data. While the quantification methods are robust and well-established methods, each of the method is associated with specific problems discussed in closer detail below:
Quantitative PCR: Quantitative PCR (qPCR), is a technique which includes the amplification of a targeted DNA molecule during the PCR, i.e. in real-time. Real-time PCR can be used quantitatively (quantitative real-time PCR), and semi-quantitatively, i.e. above/below a certain amount of DNA molecules (semi quantitative real-time PCR). Quantitative PCR (qPCR) is a gold standard of genetic target quantification. Currently, the laboratory cost of a qPCR reaction is approximately $2. However, counting in the considerable hands-on time (labour cost) for setting up the reaction, the need for standard curves, along with replicates for each quantified target, the real cost is in fact much higher. The amount of hands-on time scales steeply with an increasing number of samples since a separate quantification experiment is required for each genetic target.

Array PCR: PCR Arrays are the most reliable tools for analyzing the expression of a relevant pathway- or disease-focused panel of genes. Each 96-well plate, 384-well plate, or 100-well disc PCR Array includes SYBR Green-optimized primer assays for a thoroughly researched panel of focused panel of genes. A newer iteration of the qPCR technology is array qPCR which miniaturizes the individual qPCR reactions. Array PCR brings down the cost of an individual qPCR reaction and improves the scalability of the method to multiple targets and samples. However, the method is currently limited to profiling 384 targets from 12 samples (or conversely 12 targets from 384 samples) at a cost of thousands of dollars per chip plus a large capital cost of the read-out infrastructure. Profiling thousands of samples using the aforementioned setup, therefore, remains prohibitively expensive.

Digital PCR: Digital polymerase chain reaction (digital PCR, DigitalPCR, dPCR, or dePCR) is a method to provide absolute quantification of targets through droplet-microfluidics and fluorescent detection. The methodology is relatively cost-effective (one target per sample costs around $3), but the hands-on time for preparing, setting-up and running individual experiments for each target in each sample scales poorly to thousands of samples.

Multiplex Ligation-dependent Probe Amplification (MLPA) provides an approach to simplify the detection of multiple genetic targets in individual samples. However, MLPA provides only relative quantification of targets, and requires a separate detection experiment for each sample. More recently, a variant of MLPA introduces concepts from DNA barcoding. The concept permits a better quantitative resolution and sample multiplexing than the traditional MLPA workflow.

Next generation sequencing-based approaches: Next-generation sequencing (NGS), also known as high-throughput sequencing that makes sequence-based gene expression analysis a "digital" alternative to analog techniques. Target counting from next-generation DNA sequencing data is becoming increasingly attractive as the cost of DNA sequencing keeps decreasing, and is currently used for instance in noninvasive prenatal testing. However, the current approach suffers from high sequencing library preparation costs and sequencing efforts that is wasted on sequencing non-relevant genetic targets. For instance, in cancer-related liquid biopsies, non-targeted approaches result in wastage of sequencing effort on oncologically non-relevant loci. In fetal diagnostics, non-targeted sampling of loci considerably limits the statistical options for interpreting the data. Guardant Health Inc provides more targeted sequencing approach, where an array of RNA capture probes enriches targets for next-generation DNA sequencing.

Akhras et al. (2007) PLoS ONE 2(2):e223 disclose a multiplex pathogen detection assay involving barcoded target-specific probes, target circularization and sequencing. Use of a bridging oligonucleotide to ligate the target-specific probes is also disclosed.

WO2018109206 describes methods for the detection of analytes in a sample using padlock probes and rolling circle amplification. The use of a bridging oligo is not described.

WO2019038372 describes a next-generation sequencing approach wherein target sequences of interest are selectively amplified by in vitro transcription from ligation complexes containing a promoter for T7 polymerase, followed by cDNA synthesis and sequencing. In the method described in EP4060049 A1, rolling circle amplification from target-specific ligation complexes is utilized.

EP4332238 describes a DNA sequencing method and use for accurate and massively parallel quantification of one or more nucleic acid targets, for example in large volumes of unpurified sample material. The invention is related to a method and a kit comprising probes for detecting and quantifying genetic targets in complex samples. The invention includes two target-specific nucleic acid probes per genetic target, a barcode loop oligo and a bridge oligo or bridge oligo complex.

WO2023139309 describes a next-generation DNA sequencing-based method that utilizes target-specific nucleic acid probes and a bridge oligonucleotide or bridge oligo complex to facilitate ligation-based detection and linear amplification. The workflow includes hybridization of probes to target sequences, ligation to form ligated complexes, enzymatic removal of unligated probes, and single-directional PCR to generate linear DNA molecules. High-throughput sequencing is then employed to identify barcode sequences and determine target presence and abundance.While these methods allow accurate and parallel detection and quantification of many target sequences in a sample, more complex, large volume, dilute and/or impure samples remain challenging.

Therefore, in light of the foregoing discussion, there exists a need to overcome the aforementioned drawbacks such as, but not limited to, specificity, sensitivity, accuracy, throughput, cost, scaling and turn-over through an accurate and massively parallel quantification of nucleic acid targets.

### SUMMARY OF THE INVENTION

The present invention provides a method for amplifying specific targeted genomic material and optionally using this for next-generation sequencing for highly sensitive, scalable and accurate target quantification. The method of the present invention includes the use of a specific oligonucleotide probe complex used for molecular targeting involving a separate loop oligonucleotide for amplification, wherein the probe complex optionally includes sample indexes and/or unique molecular identifiers (UMI). The resulting method permits producing and amplifying circular molecular templates compatible with certain next-generation DNA sequencing platforms utilizing circular sequencing templates, including but not limited to, Element Biosciences Aviti or Complete Genomics G99.

In a first main aspect, the invention relates to a method for the high-throughput amplification, and optional subsequent detection, of one or more target nucleotide sequence in a plurality of samples, the method comprising the steps of:
(i) providing for each target nucleotide sequence in each of the samples:
   a first probe, a second probe, at least one loop oligo and a bridge oligo or a plurality of bridge oligonucleotides capable of annealing to the loop oligo to form a bridge oligo complex,
   wherein the first probe comprises a first bridge oligo-specific sequence at the 5' end of the first probe, optionally a first sequence barcode, and a first target specific portion at the 3' end of the first probe;
   wherein the second probe comprises a second target specific portion at the 5' end of the second probe, optionally a second sequence barcode,
   and a second bridge oligo-specific sequence at the 3' end of the second probe;
   wherein the loop oligo comprises, starting from the 5' end of the molecule, a third bridge oligo-specific sequence, a loop sequence and a fourth bridge oligo-specific sequence, wherein the loop sequence optionally comprises a third sequence barcode and wherein the loop sequence contains two sequences that are capable of annealing to each other such that a double-stranded portion is formed, wherein the double-stranded portion comprises a recognition site for a nicking endonuclease;
   wherein the bridge oligo or plurality of bridge oligonucleotides contains sequences complementary to the first bridge oligo-specific sequence and
   the second bridge oligo-specific sequence in the first probe and the second probe, respectively, and sequences complementary to the third bridge oligo-specific sequence and the fourth bridge oligo-specific sequence in the loop oligo and wherein the bridge oligo or plurality of bridge oligonucleotides optionally comprises a fourth sequence barcode;
   wherein at least one of: the first sequence barcode, the second sequence barcode, the third sequence barcode or the fourth sequence barcode is present;
(ii) contacting, for each of the one or more target nucleotide sequence, the first probe and the second probe with, preferably for each of the samples in a separate tube, the bridge oligo or plurality of bridge oligonucleotides and optionally the loop oligo, and allow self-annealing into probe complexes, wherein, if the loop oligo was added, optionally, further oligonucleotides complementary to the bridge oligo or to the plurality of bridge oligonucleotides are included to fill single-stranded gaps, if present, between the loop oligo and the first probe and/or between the loop oligo and the second probe;
(iia) if the loop oligo was added in step (ii), optionally performing polymerase extension to fill single-stranded gaps within the annealed probe complexes, if present;
(iib) if the loop oligo was added in step (ii), optionally ligating the double-stranded portions of the probe complexes,

wherein step (iib) may be performed simultaneously with step (iia);
   (iii) contacting nucleic acids present in each of the samples to be tested for the target nucleotide sequences with the probe complexes and the loop oligo, if the loop oligo was not added in step (ii);
   (iv) allowing the first target specific portion and the second target specific portion of the respective first probe and the second probe to hybridize to essentially adjacent sections on the target sequence and, if the loop oligo was added in step (iii), allowing the loop oligo to hybridize to the bridge oligo or plurality of bridge oligonucleotides, thereby forming hybridization complexes;
   (v) optionally pooling the hybridization complexes from the plurality of samples;
   (vi) ligating the probes in the hybridization complexes to provide ligated ligation complexes;
   (vii) amplifying nucleic acids from the one or more ligated ligation complexes using rolling circle amplification with a strand-displacing polymerase and allowing annealing of the two sequences in the loop sequence that are capable of annealing to each other, thereby obtaining concatemeric molecules comprising single-stranded portions and loop structures having double-stranded portions comprising the recognition sites for a nicking endonuclease;
   (viii) nicking the double-stranded portion with a nicking endonuclease having specificity for said recognition sites;
   (ix) performing a denaturation step such that the nicked concatemeric molecules disintegrate into separate segments having complementary ends; and
   (x) allowing intramolecular annealing of the complementary ends of the segments and ligating the separate segments intramolecularly, thereby obtaining circular molecules;
and, optionally, performing the additional steps of:
   (xi) subjecting the circular molecules obtained in step (x) to high-throughput sequencing technology to determine the barcode sequence(s); and
   (xii) identifying the presence and/or number of the target nucleotide sequence in the plurality of samples by determination of at least part of the first target specific portion and/or the second target specific portion, and/or at least part of the first barcode and/or the second barcode, and/or at least part of the third barcode and/or at least part of the fourth barcode,
wherein steps (v) and (vi) may be performed in any order.

### DESCRIPTION OF THE FIGURES

FIG. 1 illustrates a flow diagram of the Multiplexed Ligation Assay (MLA) according to an embodiment of the invention.
FIG. 2A, 2B, 2C, 2D, 2E, 2F, 2G, 2H and 2I illustrate principle combinations of probes according to embodiments of the invention.
FIG. 3 illustrates the effect of nicking and denaturation on the long concatemeric DNA molecules.
FIG. 4 illustrates the circularization on the monomeric DNA molecules, measured by PCR amplification through the re-ligated loop.

### DETAILED DESCRIPTION OF THE INVENTION

### Definitions:

Target Nucleotide Sequence: The term target nucleotide sequence may be any nucleotide sequence of interest of which its detection is required. It will be understood that the term given refers to a sequence of contiguous nucleotides as well as to nucleic acid molecules with the complementary sequence. The target sequence in some embodiments is a nucleotide sequence that represents or is associated with a polymorphism.

Polymorphism: The term polymorphism refers to an occurrence of two or more genetically determined alternative sequences or alleles in a population. A polymorphic marker or site is the locus at which sequence divergence occurs. A polymorphic locus may be as small as one base pair. Samples: The term samples is used herein for two or more samples which contain two or more target sequences. Samples as provided in a method according to the invention may have been prepared in order to extract at least the target nucleic acids and make those accessible to the probes as used in the invention. In particular, in some embodiments, the samples each comprise at least two different target sequences, preferably at least 100, more preferably at least 250, more preferably at least 500, most preferably at least 2000, or more. The term samples may refer to but is not limited to two or more samples obtained from a human/animal body, including urine, biopsies, saliva and other secretions, exhaled moisture extracts, tissue, blood plasma (liquid biopsies), or two or more samples obtained from the environment, including water, wastewater, soil, plants, or two or more samples containing viruses or bacteria or the like. In one embodiment, the plurality of samples includes a blood sample, a saliva sample, a urine sample or a feces sample, a sample of another body fluid or an extract from body material, for example hair or skin flakes. Probe: The term probe is a fragment of DNA or RNA of variable length (usually 50-1000 bases long, preferably 50 - 200 bases long) which can be used in DNA or RNA samples to detect the presence of nucleotide sequences (the DNA or RNA target) that are complementary to the sequence in the probe. The sections of the oligonucleotide probes that are complementary to the target sequence are designed such that for each target sequence in a sample, a pair of a first and second probe is provided, whereby the probes each contain a section at their extreme end that is complementary to a part of the target sequence. Furthermore, the present disclosure describes a bridge oligo or bridge oligo complex that is used for joining the first probe and the second probe. Moreover, the present disclosure describes a loop oligo that comprises a loop section flanked by two sections that can hybridize with the one or more bridge oligo. The loop section does not hybridize with the one or more bridge oligo and optionally comprises a barcode.

Universal: The term universal when used to describe an amplification procedure refers to a sequence that enables the use of a single primer or set of primers for a plurality of amplification reactions. The use of such primers greatly simplifies multiplexing in that only two primers are needed to amplify a plurality of selected nucleic acid sequences. The term universal when used to describe a priming site is a site to which a universal primer will hybridize. It should also be noted that "sets" of universal priming sequences/primers may be used.

Hybridization: The term hybridization (or hybridisation) describes the process of DNA or RNA molecules annealing to complementary DNA or RNA. DNA or RNA replication and transcription of DNA into RNA both rely on nucleotide hybridization.

Ligation: The term ligation is the joining of two nucleic acid fragments through the action of an enzyme. DNA ligases are enzymes capable of catalyzing the formation of a phosphodiester bond between (the ends of) two polynucleotide strands bound at adjacent sites on a complementary strand. In one embodiment, ligation can also be performed chemically, in particular if both adjacent ends of the polynucleotides are modified to allow chemical ligation.

Amplification: The term amplification as used herein denotes the use of a DNA polymerase to increase the concentration of a particular nucleotide sequence within a mixture of nucleotide sequences. "PCR" or "Polymerase Chain Reaction" is a rapid procedure for in vitro enzymatic amplification of a specific DNA/RNA segment. The DNA/RNA to be amplified may be denatured by heating the sample. The term primer is a strand of RNA or DNA (generally about 18-22 bases) that serves as a starting point for DNA synthesis. It is required for DNA replication because the enzymes that catalyze this process, DNA polymerases, can only add new nucleotides to an existing strand of DNA.

Polymerase: A polymerase is an enzyme that synthesizes long chains or polymers of nucleic acids. DNA polymerase and RNA polymerase are used to assemble DNA and RNA molecules, respectively, by copying a DNA or RNA template strand using base-pairing interactions.

High throughput: The term high throughput denotes the ability to simultaneously process and screen a large number of DNA samples; as well as to simultaneously screen large numbers of different genetic loci within a single DNA sample. High-throughput sequencing or screening, often abbreviated as HTS is a method for scientific experimentation especially relevant to effectively screen large amounts of samples simultaneously.

Endonuclease: An endonuclease is an enzyme that cleaves or nicks DNA double or single strand at a random or specified location.

Nicking endonuclease: the term nicking endonuclease has its usual meaning in the art, i.e. refers to an enzyme that cuts only one strand of a double-stranded DNA molecule to produce a DNA molecule that is nicked rather than cleaved.

Barcode: Probes and oligos used in the present invention may comprise one or more barcodes consisting of nucleotide sequences. Barcode sequences may comprise target nucleotide sequence identifier sequences, sample identifier sequences and/or molecular barcodes (also termed Unique Molecular Identifiers) for target enumeration. Barcode sequences may comprise random sequences. The terms second barcode, third barcode or fourth barcode do not necessarily mean that there is already a first or further barcodes present. As described, at least one barcode should be present that enables to uniquely define the complex within all ligation complexes in all samples tested.

As described above, the disclosure relates to a method for the high-throughput amplification and detection of target nucleotide sequences in a very large number of samples by leveraging ligation-dependent assays. The disclosure provides a method for determining the sequences of genetic targets in complex nucleic acid pools using techniques permitted by next generation sequencing. The disclosure also provides a method to profile multiple genetic targets in a number of samples, preferably a very large number of samples, by leveraging ligation-dependent assays. The disclosure provides a method for the multiplex ligation-dependent probe amplification enabling querying different target nucleic acids in a plurality of samples. The methods of the present invention allow the sequencing of the one or more target nucleotide sequence in a plurality of samples providing a plurality of different probe sets for different target nucleic acids. Unique sequence identifiers are used for the identification of the genetic targets and absolute quantification of individual samples from the sample pool when processing the sequencing data.

In a first main aspect, the invention relates to a method for the high-throughput amplification, and optional subsequent detection, of one or more target nucleotide sequence in a plurality of samples, the method comprising the steps of:
(i) providing for each target nucleotide sequence in each of the samples:
   a first probe, a second probe, at least one loop oligo and a bridge oligo or a plurality of bridge oligonucleotides capable of annealing to the loop oligo to form a bridge oligo complex,
   wherein the first probe comprises a first bridge oligo-specific sequence at the 5' end of the first probe, optionally a first sequence barcode, and a first target specific portion at the 3' end of the first probe;
   wherein the second probe comprises a second target specific portion at the 5' end of the second probe, optionally a second sequence barcode,
   and a second bridge oligo-specific sequence at the 3' end of the second probe;
   wherein the loop oligo comprises, starting from the 5' end of the molecule, a third bridge oligo-specific sequence, a loop sequence and a fourth bridge oligo-specific sequence, wherein the loop sequence optionally comprises a third sequence barcode and wherein the loop sequence contains two sequences that are capable of annealing to each other such that a double-stranded portion is formed, wherein the double-stranded portion comprises a recognition site for a nicking endonuclease;
   wherein the bridge oligo or plurality of bridge oligonucleotides contains sequences complementary to the first bridge oligo-specific sequence and
   the second bridge oligo-specific sequence in the first probe and the second probe, respectively, and sequences complementary to the third bridge oligo-specific sequence and the fourth bridge oligo-specific sequence in the loop oligo and wherein the bridge oligo or plurality of bridge oligonucleotides optionally comprises a fourth sequence barcode;
   wherein at least one of: the first sequence barcode, the second sequence barcode, the third sequence barcode or the fourth sequence barcode is present;
(ii) contacting, for each of the one or more target nucleotide sequence, the first probe and the second probe with, preferably for each of the samples in a separate tube, the bridge oligo or plurality of bridge oligonucleotides and optionally the loop oligo, and allow self-annealing into probe complexes, wherein, if the loop oligo was added, optionally, further oligonucleotides complementary to the bridge oligo or to the plurality of bridge oligonucleotides are included to fill single-stranded gaps, if present, between the loop oligo and the first probe and/or between the loop oligo and the second probe;
(iia) if the loop oligo was added in step (ii), optionally performing polymerase extension to fill single-stranded gaps within the annealed probe complexes, if present;
(iib) if the loop oligo was added in step (ii), optionally ligating the double-stranded portions of the probe complexes,

wherein step (iib) may be performed simultaneously with step (iia);
   (iii) contacting nucleic acids present in each of the samples to be tested for the target nucleotide sequences with the probe complexes and the loop oligo, if the loop oligo was not added in step (ii);
   (iv) allowing the first target specific portion and the second target specific portion of the respective first probe and the second probe to hybridize to essentially adjacent sections on the target sequence and, if the loop oligo was added in step (iii), allowing the loop oligo to hybridize to the bridge oligo or plurality of bridge oligonucleotides, thereby forming hybridization complexes;
   (v) optionally pooling the hybridization complexes from the plurality of samples;
   (vi) ligating the probes in the hybridization complexes to provide ligated ligation complexes;
   (vii) amplifying nucleic acids from the one or more ligated ligation complexes using rolling circle amplification with a strand-displacing polymerase and allowing annealing of the two sequences in the loop sequence that are capable of annealing to each other, thereby obtaining concatemeric molecules comprising single-stranded portions and loop structures having double-stranded portions comprising the recognition sites for a nicking endonuclease;
   (viii) nicking the double-stranded portion with a nicking endonuclease having specificity for said recognition sites;
   (ix) performing a denaturation step such that the nicked concatemeric molecules disintegrate into separate segments having complementary ends; and
   (x) allowing intramolecular annealing of the complementary ends of the segments and ligating the separate segments intramolecularly, thereby obtaining circular molecules;
and, optionally, performing the additional steps of:
   (xi) subjecting the circular molecules obtained in step (x) to high-throughput sequencing technology to determine the barcode sequence(s); and
   (xii) identifying the presence and/or number of the target nucleotide sequence in the plurality of samples by determination of at least part of the first target specific portion and/or the second target specific portion, and/or at least part of the first barcode and/or the second barcode, and/or at least part of the third barcode and/or at least part of the fourth barcode,
wherein steps (v) and (vi) may be performed in any order.

In one embodiment, the method comprises steps (xi) and (xii).

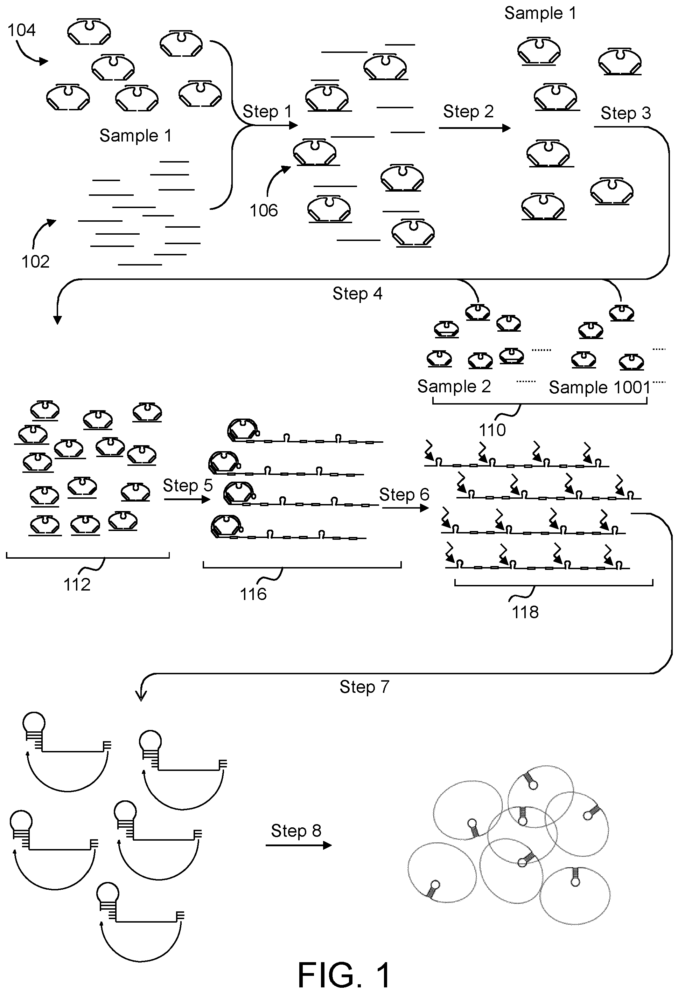

Figure 1 provides a non-limiting illustration of an embodiment of the method of the invention.

The methods of the present invention utilize four or more nucleic acid molecules, out of which two target-specific nucleic acid probes (first probe and second probe) are specific for a genetic target and two or more other nucleic acid probes typically are universal (bridge oligo or bridge oligo complex and loop oligo). The first probe, second probe and loop oligo hybridize to the one or more bridge probe forming a probe complex. The probe complexes (containing one or more barcode sequences) having target identification sites on the sample DNA or RNA are allowed to hybridize against complementary target sequences of the query sample. After hybridization, the first and second probe are ligated chemically or enzymatically by a DNA ligase to form ligated ligation complex. In the present invention, a plurality of such ligated ligation complexes will form during the sample analysis in the plurality of samples to be analyzed.

A "plurality of samples" may refer to, but is not limited to, two or more samples obtained from the human or animal body, including biopsies, saliva and other secretions, exhaled moisture extracts, tissue, blood plasma (liquid biopsies), two or more samples obtained from environment, including water, wastewater, soil, plants, or two or more samples containing viruses or bacteria or the like. In one embodiment, the sample is used without any prior purification or concentration of nucleic acid. In another embodiment, the sample may be pre-treated, for instance lysing cells to expose nucleic acid.

The target sequence may include any nucleotide sequence of interest against which the detection is required. The target nucleotide sequence of the disclosure may be obtained from, but not limited to, a fraction of DNA in the patient's blood or a fraction of DNA in maternal blood. A fraction of the DNA in the patient's blood may for example be obtained from apoptotic/necrotic cancer cells or a fraction of DNA in maternal blood from fetal and/or maternal origin. Further, the results of the analysis are used to, for instance, assess the risk of an individual to a given type of cancer, to determine the efficacy of a given treatment against a given cancer, the development of a drug-resistance-related mutations in a tumor, or the risk of a fetus carrying genetic disorders such as common trisomies Down, Patau and Edwards syndromes. In certain embodiments, the method comprises providing, for each target nucleotide sequence, a plurality of different probe sets.

As used herein, the term probe sets includes a first probe, a second probe, loop oligo and one or more bridge oligo.

In certain embodiments, the first probe includes, starting from the 5' end of the molecule, optionally a 5' phosphate, a first bridge oligo-specific sequence, optionally a first universal sequence, optionally a first sequence barcode, and a first target specific portion at its 3' end.

In certain embodiments, the second probe includes, starting from 5' end of the molecule, optionally a 5' phosphate, a second target specific portion, optionally a second sequence barcode, optionally a second universal sequence, and a second bridge oligo-specific sequence at its 3' end.

The bridge oligo or plurality of bridge oligos contains sequences complementary to the first and second bridge oligo-specific sequences in the first and second probe, respectively, optionally a universal sequence, optionally a fourth sequence barcode, and sequences complementary to the third bridge oligo-specific sequence and the fourth bridge oligo-specific sequence in the loop oligo.

The loop oligo comprises starting from the 5' end of the molecule, a third bridge oligo-specific sequence, a loop sequence and a fourth bridge oligo-specific sequence, wherein the loop sequence optionally comprises a third sequence barcode and wherein the loop sequence contains two sequences that are capable of annealing to each other such that a double-stranded portion is formed, wherein the double-stranded portion comprises a recognition site for a nicking endonuclease. The two sequences that are capable of annealing to each other contained within the loop sequence may each have a length of at least 5 bases, such as at least 6, 7, 8, 9 or at least 10 bases, for example a length of between 10 and 50 bases, such as between 10 and 25 bases or between 10 and 15 bases. The barcodes may be used to enable to uniquely define the complex within all ligation complexes in all samples tested. The loop oligo may have any suitable length and may, for example, be between 30 and 100 bp in length, such as between 40 and 60 bp in length.

Optionally, at least one of: the first probe, the second probe, the loop oligo or the one or more bridge oligo comprises a first capture moiety. A first capture moiety, when used herein, refers to a moiety, such as a chemical group, which allows the probe, probe complex, ligation complex or hybridization complex to be captured by, i.e. bound to, a second capture moiety which is linked to a solid support. Any suitable capture moiety known in the art may be used for this purpose. A well-known suitable example is the capture of biotinylated molecules using streptavidin-coated magnetic beads. Thus, in one embodiment, the first capture moiety is a biotin moiety, which can interact with a streptavidin or avidin moiety (the second capture moiety) linked to a solid support, such as a magnetic bead. Other options include biotin derivatives such as dual-biotin, desthiobiotin or photocleavable biotin which can be used for conjugation with streptavidin/avidin. Further options include the use of thiol and acrydite groups for acrydite/acrylamide conjugation, alkyne and azide groups for click chemistry and digoxigenin for anti-digoxigenin antibody conjugation. The conjugation partners can be provided on any solid surfaces such as beads (magnetic or otherwise) or solid supports. Accordingly, in one embodiment of the method of the invention, at least one of: the first probe, the second probe, the loop oligo or the one or more bridge oligo comprises a first capture moiety, and between steps (iv) and (v) an intermediate step (iv)(a) is performed which comprises bringing the hybridization complex(es) in contact with a solid support comprising a second capture moiety, allowing the first capture moiety and the second capture moiety to interact such that the hybridization complexes become linked to the solid support and separating the solid-support-linked hybridization complexes from components of the samples that are not linked to the solid-support.

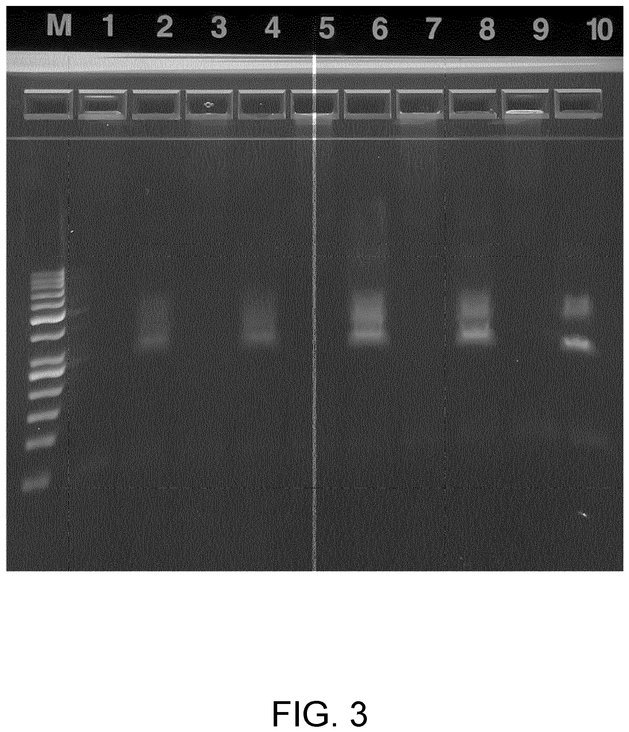

The first target specific portion, the second target specific portion, the first bridge oligo-specific sequences, the second bridge oligo-specific sequences, the third bridge oligo-specific sequences and/or the fourth bridge oligo-specific sequences, optionally contain independently from one another at least one chemically modified nucleotide to increase probe binding. The chemical modifications that increase probe binding include, but are not limited to, ribonucleic acids, peptide nucleic acids, and locked nucleic acids (e.g. as illustrated in Figure 3 of WO2019038372). In one embodiment, the bridging portion of the first probe, the second probe, the loop oligo or all three of these, comprise(s) chemically modified bases to permit improved binding to the one or more bridge oligo. In another embodiment, the first target specific portion, the second target specific portion, the first bridge oligo-specific sequences, the second bridge oligo-specific sequences, the third bridge oligo-specific sequences and/or the fourth bridge oligo-specific sequences contain independently from one another, one or more chemically modified nucleotides. In certain embodiments, chemical modifications permit chemical ligation of adjacent probes. In one embodiment, one or more of: the first probe, the second probe, the loop oligo, the bridge oligo or an oligonucleotide of the plurality of bridge oligonucleotides is modified to permit chemical ligation.

In some embodiments, the aforementioned probes bind to completely adjacent genetic loci, i.e. adjacent sections of the target nucleotide sequence, or up to 500 base pairs apart, for example up to 200 base pairs apart, such as up to 50 base pairs apart, up to 40 base pairs apart, up to 30 base pairs apart, up to 20 base pairs apart up to 10 base pairs apart or up to 5 base pairs apart.

In some embodiments, the first probe, the second probe, the loop oligo or the one or more bridge oligo may include adapter sequences for a DNA sequencing platform such as (but not limited to) Illumina, Element Biosciences, Complete Genomics or Pacific Biosciences platforms. These adapter sequences permit the resulting sequencing libraries to bind to the detection parts of the sequencing devices such as Illumina, Element Biosciences, Complete Genomics or Pacific Biosciences flow cells.

Furthermore, in some embodiments, the bridge oligo or an oligonucleotide of the plurality of bridge oligonucleotide comprises:
(i) one to five 3' protruding bases (i.e. additional bases which do not form double helix with the second probe), and/or
(ii) 3' phosphate, and/or
(iii) one or more phosphorothioate modifications within three positions from the 3' end, and/or
(iv) another modification that protects the bridge from exonuclease activity and/or prevent amplification from the 3' end.

Before contacting the probes with the sample comprising the target sequences, the first probe, the second probe and optionally the loop oligo are brought in contact with the bridge oligo (or plurality of oligonucleotides capable of forming a bridge oligo complex), preferably for each of the samples in a separate tube, and annealing into probe complexes is allowed (step (ii)). The bridge oligo and the loop oligo may be pre-annealed before annealing with the first and second probes or all annealing steps may be done at once. In an embodiment wherein the bridge is not one oligo, but a plurality of oligonucleotides, such as two oligonucleotides, capable of annealing to the loop oligo to form a bridge oligo complex (illustrated herein in 2G), the plurality of oligonucleotides may be pre-annealed with the loop oligo before annealing with the first and second probes or all annealing steps may be done at once.

In some embodiments, if the loop oligo was added in step (ii), the probes and oligonucleotides in the probe complex may be ligated prior to proceeding with step (iii).

In some embodiments, if the loop oligo was added in step (ii), if single-stranded gaps remain when the first probe, second probe and loop are annealed to the bridge oligo or plurality of bridge oligonucleotides, such gaps may be filled. This may be performed by including further oligonucleotides complementary to the bridge oligo or to the plurality of bridge oligonucleotides. Alternatively, or in addition, a polymerase extension may be performed to fill single-stranded gaps within the annealed probe complexes. Following, or simultaneously, with gap filling, a ligase may be added to ligate double-stranded portions of the probe.

Preferably each probe complex is unique for the combination of the first target specific sequence, the second target specific sequence and one or more barcode sequences. This enables enumeration of the target sequences after amplification and analysis of the results.

Thereafter, in step (iii), one or more target nucleotide sequences in the plurality of samples is brought into contact with the plurality of probe complexes and the loop oligo, if the loop oligo was not added in step (ii). Thus, the method of the invention includes the use of a loop oligo, which may be added in step (ii) or in step (iii). In one embodiment, the loop oligo is added in step (ii). In another embodiment, the loop oligo is added in step (iii). Contacting the probe complexes with the samples and the loop oligo in step (iii), if the loop oligo is added in step (iii), may be done simultaneously or sequentially in any order, i.e. first contacting with the samples and then with the loop oligo or first contacting with the loop oligo and then with the samples.

The first target specific portion and the second target specific portion of the respective first probe and the second probe hybridize to essentially adjacent sections on the target sequence, and, if the loop oligo was added in step (iii), allowing the loop oligo to hybridize to the bridge oligo or plurality of bridge oligonucleotides, thereby forming a hybridization complex (step (iv)). It is to be understood that in the context of step (iv), reference to the first probe, the second probe and the bridge oligo or plurality of bridge oligonucleotides, is intended to refer to those parts of the probe complex that are derived from these probes or oligos. As mentioned above, the essentially adjacent sections on the target sequence may be immediately adjacent or there may be a gap of up to 500 base pairs apart.

In some embodiments, the sample has a volume of more than 100 microliters, e.g. more than 1 ml. In a further embodiment, the sample has a nucleic acid concentration below 5 pmol, such as below 1 pmol, for example below 200 fmol. In one embodiment, the plurality of samples includes one or more blood samples, one or more saliva samples, one or more urine samples or one or more feces samples.

Subsequently, in some embodiments, if at least one of: the first probe, the second probe, the loop oligo or the one or more bridge oligo comprises a first capture moiety, the hybridization complex(es) are brought in contact with a solid support comprising a second capture moiety and the first capture moiety and the second capture moiety are allowed to interact such that the hybridization complex(es) become linked to the solid support (optional step (iv)(a)). Thereafter, the solid-support-linked hybridization complexes are separated from components of the samples that are not linked to the solid-support. If the solid supports are magnetic beads, the beads may be immobilized using a magnet and the remaining liquid sample may be removed. Optionally, a wash step is performed before proceeding.

Step (iv)(a) results in a purification and enrichment for nucleic acid, allowing improved results in particular for highly impure samples. In one embodiment, the method of the invention does not comprise a step of enriching for nucleic acids prior to step (iv)(a). Thus, in one embodiment, the method does not contain prior to step (vi) a step wherein nucleic acids in the original sample are concentrated more than 2-fold, more than 10-fold, or more than 100-fold. In another embodiment, the method of the invention does not include a purification step subsequent to the ligation in step (vi).

Subsequently, ligation of the probes in the formed hybridized complexes is carried out either enzymatically or chemically to provide ligated ligation complexes (step (vi)). Optionally as a part of step (vi), a gap between the first probe and the second probe, between the first probe and the loop oligo and/or between the second probe and the loop oligo, if present, may be filled by introducing a polymerase and one or more nucleotides. The polymerase adds nucleotides (a) complimentary to the bridge oligo sequence(s) and thereby fills in the gaps between the first probe, the second probe and the loop oligo resulting in ligated the probes and inclusion of the loop oligo into the bridge complementary strand. The bridge oligo or bridge oligo complex is extended from the 5' site or the 3' site complimentary to the ligated probes such that the target sequence identifier sequence present in the first probe or second probe is integrated into the bridge oligo or bridge oligo complex. Preferably, a polymerase is used that does not break up double stranded DNA, such as for instance a Taq polymerase, in order not to interfere with the ligation of the first to the second probe when both are annealed to the target sequence. In one embodiment, the bridge oligo, or one or more oligonucleotides of the plurality of bridge oligonucleotides, comprises, in a region not complementary to the first probe, the second probe or the loop oligo, a plurality of universal base analogues to permit the incorporation of random sequences suitable for use as molecular barcode for target enumeration. In such an embodiment, as part of step (vi), a gap filling step is performed using polymerase and nucleotides in order to generate such random sequences. In embodiment, plurality of universal base analogues is a plurality of 5-nitroindoles.

The ligated ligation complexes are then optionally pooled from one or more target samples (step (vi)). Steps (v) and (vi) may be performed in the specified order or alternatively in reverse order.

Next, nucleic acids are amplified from the one or more ligated ligation complexes, thereby obtaining single-stranded concatemeric sequences (step (vii)). Amplification is performed using rolling circle amplification with a strand-displacing polymerase, such as phi29 polymerase (UniProtKB - P03680; DPOL_BPPH2) or a Bst polymerase (P52026; DPO1_GEOSE) starting from the 3' end of the bridge oligo and/or a target-specific probe or starting from a universal oligo complementary to a conserved part of the ligated ligation complex added immediately prior to step (vii) and annealing of the two sequences in the loop sequence that are capable of annealing to each other is allowed to take place, thereby obtaining concatemeric molecules comprising single-stranded portions and loop structures having double-stranded portions comprising the recognition sites for a nicking endonuclease. In a further embodiment hereof, one or more additional oligonucleotide having complementarity to the amplifying concatemer may be added to permit exponential branching amplification.

In one embodiment, subsequent to step (vi), but prior to step (vii), a step (a) and a step (b) are performed, wherein step (a) comprises allowing the ligated ligation complexes to dissociate from the target nucleotide sequence and step (b) comprises adding a target-specific probe comprising a sequence corresponding to the target nucleotide sequence, wherein said target-specific probe is capable of annealing with the ligated ligation complexes, and allowing the target-specific probe to anneal to the ligated ligation complexes thereby forming amplification templates. In such an embodiment, said amplification templates are amplified by rolling circle amplification with a strand-displacing polymerase in step (vii).

Optionally, after amplification in step (vii), the solid supports, if present, are removed and the supernatant is used for subsequent processing. For example, if the solid supports are magnetic particles, these may be removed using a magnet. In some other embodiments of the method of the invention, the interaction between the first capture moiety and the second capture moiety is disrupted immediately after step (v), after step (vi) or after step (vii). For example, if the first capture moiety is biotin and the second capture moiety is streptavidin, the interaction can be disrupted by adding excess soluble biotin. If the streptavidin is bound to magnetic particles, it can subsequently be removed using a magnet.

Subsequent to step (vii), in step (viii), the double-stranded portions of the loop structures are nicked with a nicking endonuclease having specificity for said recognition sites. In one embodiment, the nicking endonuclease is Nb.BbvCI or Nb.BsrDI.

Step (ix) comprises performing a denaturation step such that the nicked concatemeric molecules disintegrate into separate segments having complementary ends. Said denaturation may be performed using methods well-known in the art for the denaturation of double stranded DNA, such as heating and/or treatment with chemicals.

Subsequent step (x) comprises allowing intramolecular annealing of the complementary ends of the segments and ligating the separate segments intramolecularly, thereby obtaining circular molecules.

Optionally, the method comprises performing the additional steps of:
(xi) subjecting the circular molecules obtained in step (x) to high-throughput sequencing technology, including but not limited to, platforms provided by Element Biosciences or Complete Genomics to determine the barcode sequence(s); and
(xii) identifying the presence and/or number of the target nucleotide sequence in the plurality of samples by determination of at least part of the first target specific portion and/or the second target specific portion, and/or at least part of the first barcode and/or the second barcode, and/or at least part of the third barcode and/or at least part of the fourth barcode.

The identification of the presence and/or number of the target nucleotide sequence in the plurality of samples may be performed by determination of at least part of the first and/or second target specific portion, and/or at least part of a barcode by high-throughput sequencing technology (steps (xi) and (xii)), for example using a next-generation sequencing platform including without limiting, Element Biosciences Aviti or Complete Genomics G99. Preferably, the genetic target enumeration is permitted by counting the number of molecular barcodes or sequencing reads per target and per sample. The samples are separated (de-convoluted) from the sequence data and the sequence targets quantified in silico after the DNA sequencing.

The advantages of the present invention include, but are not limited to quantification assay with low cost, high simplicity, high specificity, high sensitivity, high accuracy, high throughput, high scalability and high turn-over in comparison to traditional nucleic acid sequencing technologies. Another aspect of the present invention is that the methods of the present invention allow multiple samples to be pooled due to sample indexing at an initial stage of the workflow, providing improvements in assay costs and speed. Another aspect of the present invention is that the methods of the present invention allow accurate and massively parallel quantification of plurality of nucleic acid targets in multiple samples including human and animal populations, and including large volumes of unpurified sample material. As mentioned, in a preferred embodiment, the sample, such as a urine sample, is used without any prior purification or concentration of nucleic acid. In another embodiment, the sample may be pre-treated, for instance lysing cells to expose nucleic acid. One particular advantage of the invention is to enable the detection and amplification of target sequence of interest using unique probe designs, i.e., probe triplet. The probes are designed with specially situated modified nucleotides that improve annealing and binding efficiency. Improvement in binding properties leads to higher assay specificity, sensitivity and accuracy. The methods of the present invention are likewise applicable for studying genetic variants and find application in diagnosis and prognosis, including but not limited to genotype the sample(s) for one or more sequences and/or polymorphisms, such as SNPs and/or indels, cancer diagnostics or fetal chromosomal disorders from maternal blood. In a preferred embodiment, for two or more samples or for two or more locus/allele combinations, barcode sequences are used to genotype the samples for one or more sequences and/or polymorphisms, such as SNPs and/or indels.

One particular advantage of the invention is to enable the detection and amplification of target sequence of interest using unique probe designs. The probes are designed with improved binding properties lead to higher assay specificity, sensitivity and accuracy. The present invention finds application in the area of molecular biology, evolutionary biology, metagenomics, genotyping and more specifically, but not limited to cancer diagnostics or fetal chromosomal disorders, including but not limited to genotype sample(s) for one or more sequences and/or polymorphisms, such as SNPs and/or indels.

In one particular preferred embodiment, the loop oligo comprises information to identify the sample and includes a unique barcode. In such case, the first and second probe is universally applicable to all samples (and only comprises information to identify the target). In one preferred embodiment, therefore, a method according to the invention is provided, wherein the loop oligo comprises a barcode that comprises a unique sequence that enables enumeration of the target sequences of each sample.

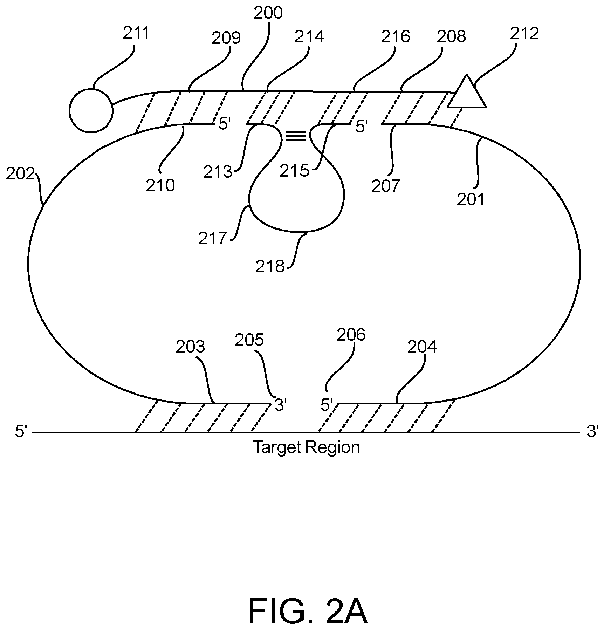

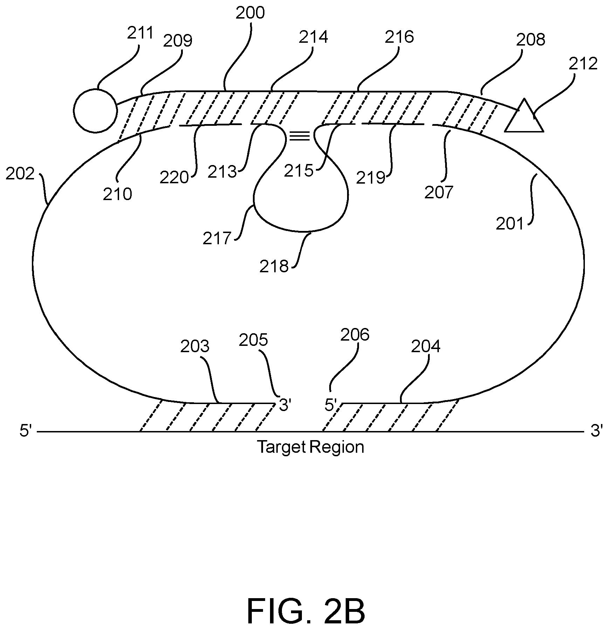

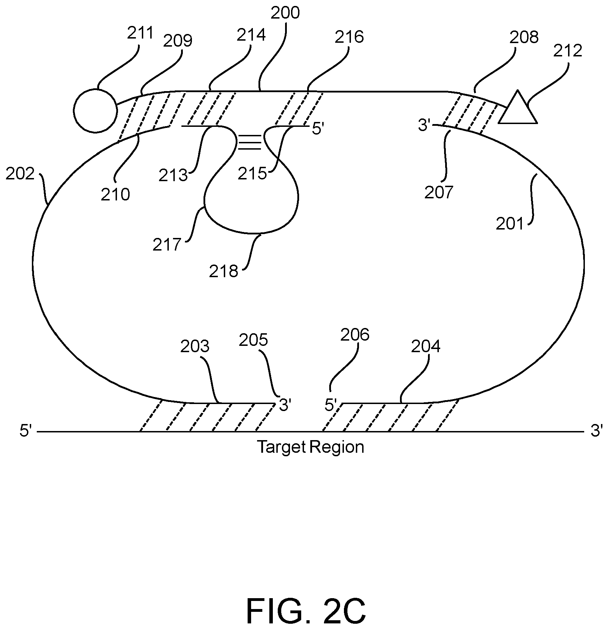

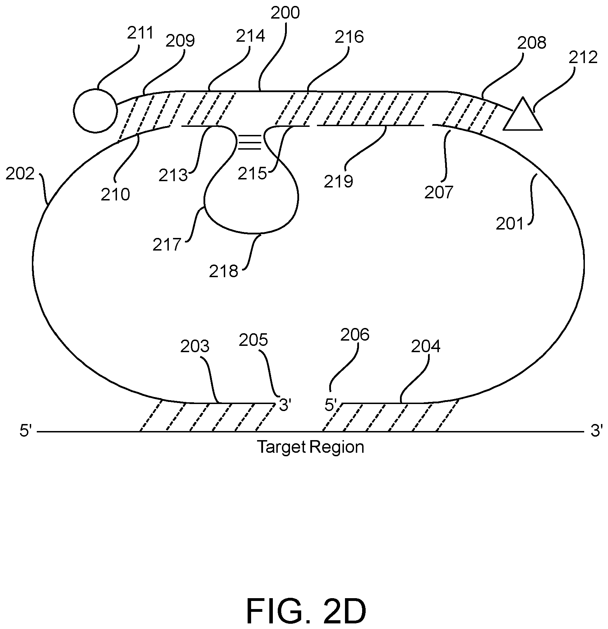

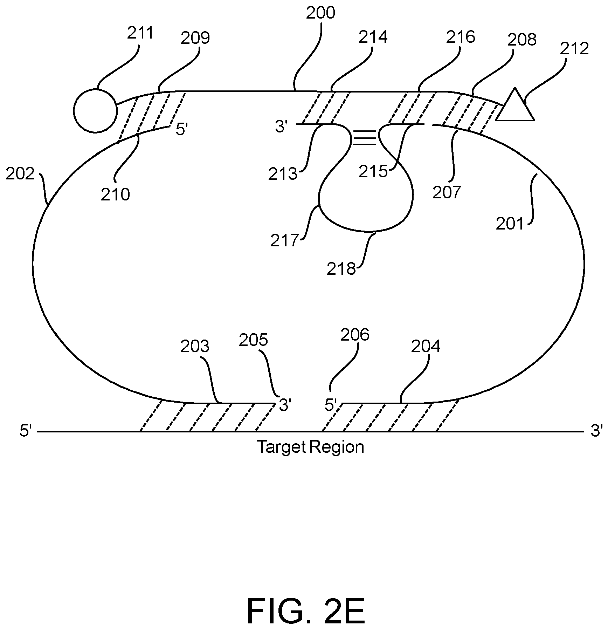

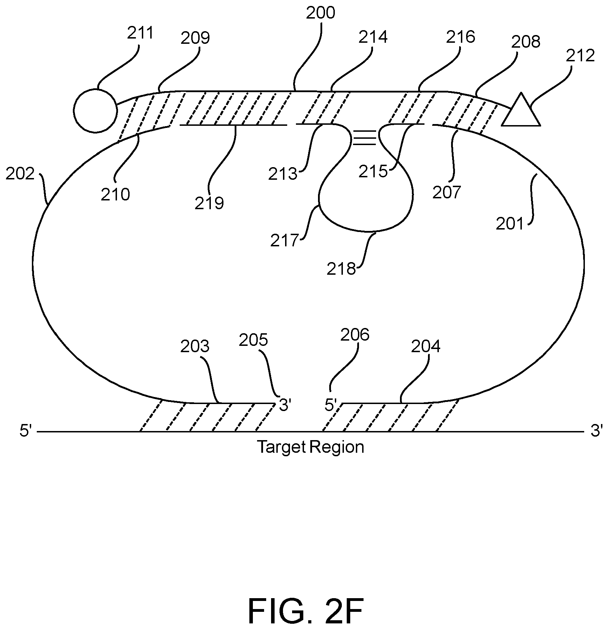

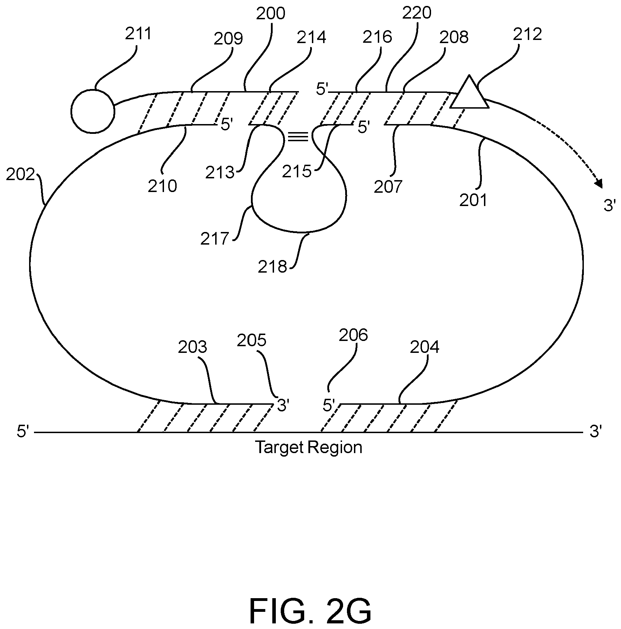

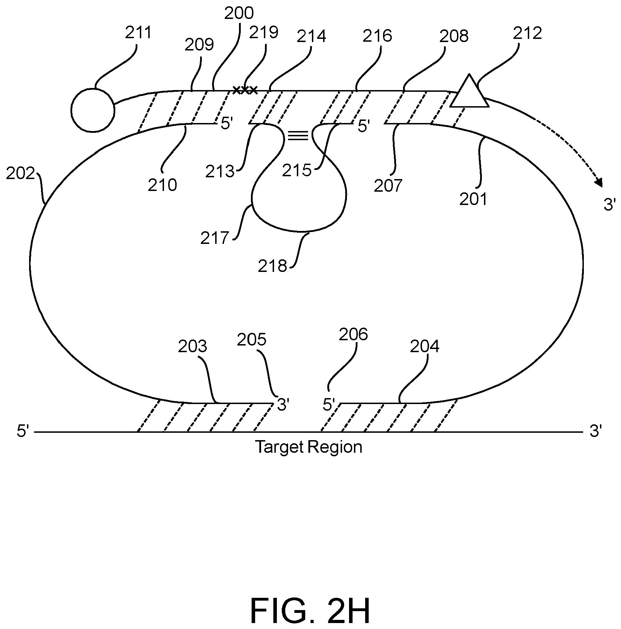

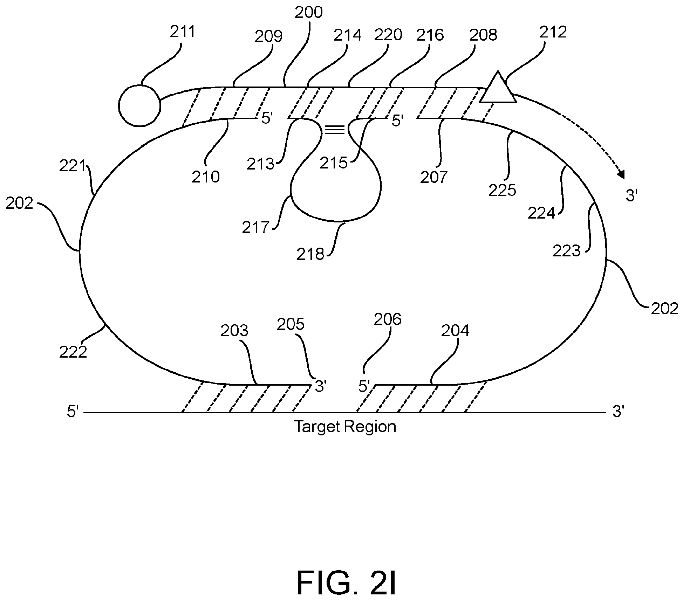

The following Examples and Figures 1 and 2 are included to illustrate the invention and are not intended to be limiting.

### EXAMPLES

### Method

### 1. Formation of probe complexes

Probe complexes contain sequences required for genomic targeting, sample indexing and circle amplification.

Four-part probe complexes are allowed to form (as illustrated in Figure 2), comprising:
(a) a first probe having, starting from the 5' end of the molecule, a first bridge oligo-specific sequence, and a first target specific portion at the 3' end of first probe;
(b) a second probe having, starting from the 5' end of the molecule, a second target specific portion, a second sequence barcode, and a second bridge oligo-specific sequence at the 3' end of second probe;
(c) a bridge oligo having sequences complementary to the first bridge oligo-specific sequence and the second bridge oligo-specific sequence in the first probe and the second probe, respectively; and
(d) a loop oligo having sequences complementary to the bridge oligo.

Probe complexes are built by combining all three parts (bridge, right arm and left arm) in equimolar amounts in annealing reaction. The reaction is carried out in a thermo cycler (Annealing program in Table 1).

**Table 1.**

| **Step** | **Temperature** | **Time** |
|---|---|---|
| **1** | +95°C | 5 min |
| **2** | +95°C | 1 min |

| -4°C/cycle, go to 2 10x | | |
|---|---|---|
| **3** | +55°C | 10 min |
| **4** | +55°C | 1 min |

| -5°C/cycle, go to 5 5x | | |
|---|---|---|
| **5** | +4°C | hold |

### 2. Target capture

Specific genomic regions containing the mutation(s) of interest are targeted. Purified DNA (for example from tissue, plasma, urine or saliva) can be used as sample or the samples can be non-purified, but only preprocessed, for example by boiling and/or centrifugation.

The probe complexes are hybridized to target regions via base sequence complementary interactions. To initiate the target capture, the probe complexes and target DNA are mixed and incubated in a thermal cycler (Target capture and GapFill program in Table 2).

**Table 2.**

| **Step** | **Temperature** | **Time** | **Process** |
|---|---|---|---|
| 1 | +95°C | 4 min | Denaturation |
| 2 | +85°C | 2,5 min | |
| 3 | +75°C | 2,5 min | |
| 4 | +65°C | 2,5 min | |
| 5 | +55°C | 120 min | target capture |
| 6 | +50°C | 10 min | GapFIII |
| 7 | +45°C | 45 min | |
| 8 | +4°C | hold | |

### 3. GapFill reaction

After target capture, the probe complexes from separate targeting reactions are pooled and subsequently extended and ligated by adding a combination of Phusion DNA polymerase, nucleotides and Ampligase DNA ligase and incubating 30 minutes at +55°C.

### 4. Exonuclease treatments

After GapFill linear molecules are removed by adding 1µl of Thermolabile Exonuclease 1 (NEB, #M0568L) and 1µl of RecJF Exonuclease (NEB, #M0264L) and incubating 30 minutes in +37°C. Exonucleases are inactivated by incubating 10 minutes in +92°C.

### 5. Purification of circle molecules

After exonuclease treatment the circular probe molecules are purified with size selection beads (such as Becham Coulter AMPure XP or Macherey Nagel NucleoMag). Purity and quantity of circle probe molecules is determined by qPCR or some equivalent method.

### 6. Rolling Circle Amplification

After purification, circular probe molecules are subjected to Rolling Circle Amplification (RCA). For RCA reaction circular probe molecules are mixed with RCA reaction mix containing EquipPhi29 (Thermo Scientific) polymerase and specific oligonucleotide primer. Reaction is incubated at +42°C for 30 min - 2 hours. After RCA reaction the efficiency of reaction is analyzed by measuring the concentration of single stranded DNA (ssDNA) with Qubit fluorometer or qPCR.

### 7. Loop folding

After Rolling circle amplification (RCA) reaction long concatemeric ssDNA molecule having multiple copies of target library and loop oligo is subjected to folding reaction. Reaction conditions are adjusted by supplementing buffer with folding modifiers such as (including but not limited to MgCl2 and dendrimers such as polyamidoimine (PANAM), polypropylenimine (PPI), polyethyleneimine (PEI)). Reaction is incubated in +95°C for 6 minutes and then incrementally cooled down to +25°C within 30 minutes.

### 8. Nicking reaction/Loop digestion

Loop folding reaction produces a long concatemeric ssDNA molecule having multiple copies of precisely located dsDNA loops separating each complete target library segment. Looping forms double stranded recognition sequence for specific nicking endonuclease (including but not limiting to Nb.BbvCI or Nb.BsrDI) This sequence permits sequence-specific cutting of the long concatemer to separate target library segments having complementary ends. RCA products are digested with nicking endonucleases for 30 to 60 min at +37°C. Nicking endonucleases are inactivated by incubating 8 minutes in +80°C. Efficiency of nicking reaction is measured with qPCR or agarose gels.

### 9. Purification of target library molecules

After nicking endonuclease treatment the target library molecules are purified with size selection beads (such as Beckman Coulter AMPure XP or Macherey Nagel NucleoMag). Purity and quantity of molecules is determined with qPCR or similar methods.

### 10. Re-ligation of target library molecules

After purification, the molecule ends are intramolecularly ligated to form circular ssDNA molecules. Purified molecules are supplemented with reaction buffer and ligated with T4 DNA ligase for 90 min in +26°C. After ligation, non-circularized molecules are removed by adding 1µl of Thermolabile Exonuclease 1 (NEB, #M0568L) and 1µl of RecJF Exonuclease (NEB, #M0264L) and incubating 30 minutes in +37°C. Reactions are inactivated by incubating 10 minutes in +92°C. Purity and quantity of molecules is determined with qPCR or equivalent methods.

### 11. Purification of circular molecules

After ligation the target library molecules are purified with size selection beads (such as Becham Coulter AMPure XP or Macherey Nagel NucleoMag). Purity and quantity of molecules is determined with qPCR or equivalent methods.

### Results

FIG. 3 illustrates the cleavage of the concatemeric DNA molecules using a nicking endonuclease. The lanes with odd numbers represent non-nicked concatemeric DNA molecules and the lanes with even numbers represent DNA molecules originating from the concatemer after nicking and denaturation. The non-nicked concatemeric DNA molecules do not migrate far from the loading well on top of the electrophoretic gel due to their large size while nicking and denaturation of the concatemeric DNA molecules results in small molecules which easily migrate down on the electrophoretic gel.

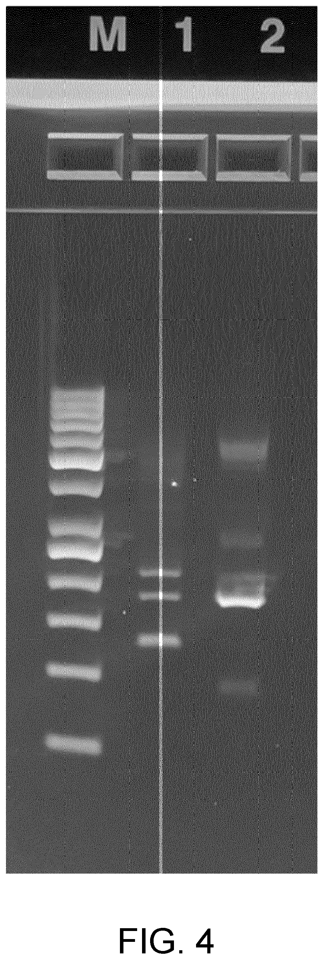

FIG. 4 illustrates the circularization of the monomeric DNA molecules resulting from the nicking and denaturation of the concatemeric DNA molecules. The circularization is measured by PCR amplification through the re-ligated loop, resulting in a bright amplification product of the correct size on lane 2.

### DETAILED DESCRIPTION OF FIGURES 1 AND 2

FIG 1 illustrates the workflow of one embodiment of the described invention. In step 1, nucleic acids (DNA or RNA) within a sample (102) are brought into contact with a set of probe complexes (104). The probe complexes anneal on the target nucleic acids (106). In step 2, the target-bound probe complexes are optionally captured from the sample material. In step 3, ligated probe complexes from multiple samples (110) are pooled together (112). In step 4, the annealed, pooled ligation complexes are ligated, resulting in ligated ligation complexes. In step 5, the probe sequences are amplified by rolling circle amplification using phi29 polymerase or other strand displacing polymerase, resulting in long concatemeric copies of the probes (116). In step 6, the concatemeric probe copies are cleaved into nicked concatemeric DNA using a nicking endonuclease such as Nb.BbvCI or Nb.BsrDI. In step 7, the nicked concatemeric DNA is denatured to break the concatemeric structure into monomeric units and the monomeric units are renatured into nicked circular DNA molecules. In step 8, the nicked circular DNA molecules are ligated into circular DNA molecules, which optionally can be used as templates for sequencing.

FIG. 2A illustrates a principle structure of a probe quadruplet according to an embodiment herein. The plurality of probe entities includes a first probe (202), a second probe (201), a bridge oligo (200) and a loop oligo (217). Here the probe complex contains gaps or nicks between the first probe and the loop oligo (210 and 213), between the second probe and the and the loop oligo (207 and 215) and between the target-specific parts of the first and second probes (203 and 204). These gaps are filled by introducing a polymerase and one or more nucleotides. For this process, a mixture of Stoffel fragment, Taq polymerase or Phusion polymerase, and DNA ligase such as Ampligase can be used. The polymerase fills these gaps and the subsequent action of the DNA ligase results in ligation of the probe, bridge and loop oligos into a circular complex.

15-25 bases of the first probe includes a bridge binding sequence (210), that optionally includes chemically modified bases for efficient bridge oligo binding. The first probe further includes 15-30 bases from the 5' end, binding to the genetic target (203). Some or all of the nucleotides of 210 may include chemical modifications that increase the affinity of the probes to the target or the bridge (209). The last base of the first probe optionally includes a phosphate moiety for enzymatic ligation or a modification that permits chemical ligation to the 5' end of the adjacent probe (205).

The first base of the second probe optionally includes a phosphate moiety for enzymatic ligation or a modification that permits chemical ligation to the 5' end of the adjacent probe (206). The 15-30 bases from the 5' end of the second probe include a part of the second probe that binds to the genetic target (204). The last 15-25 bases of the second probe (207), are reverse complementary to the bridge oligo (208). Some or all of the nucleotides of 203, 204, 207 or 210 may include chemical modifications that increase the affinity of the probes to the target or the bridge oligo.

The first 15-25 bases from the 5' end of the bridge oligo (209), are reverse complementary to the bridge-oligo specific sequence of the first probe (210), and optionally include chemically modified nucleotides for increased binding. The last 15-25 bases of the bridge oligo (208), are reverse complementary to the sequence of the second probe (207), and optionally include chemically modified nucleotides for increased binding. The 5' end of the bridge oligo optionally includes a capture moiety (211) used for capturing the probe complexes. Furthermore, the bridge oligo comprises sequences 214 and 216, complementary to sequences 213 and 215 of the loop oligo. The 3' end of the bridge oligo optionally includes a phosphate (or other cleavable) moiety (212) to prevent extension during gap fill.

The first 15-25 bases from the 5' end of the loop oligo (215) are reverse complementary to bridge oligo sequence 216. The loop oligo optionally comprises a barcode (218). The last 15-25 bases of the loop oligo (213) are reverse complementary to bridge oligo sequence 214.

The first probe, the bridge oligo, the loop oligo and/or the second probe may contain barcode sequences such as sample barcodes or unique molecular identifiers.

FIG. 2B illustrates a principle structure of a probe set having a plurality of probe entities according to an embodiment of the invention. The probes correspond to those of FIG. 2A, except that this embodiment comprises two additional oligonucleotides aligned to the bridge oligo around the loop oligo (219 and 220), thus leaving nicks between 210 and 220, 220 and 213, 215 and 219, and 219 and 207, and permitting joining the first probe (202), the first additional oligo (220), the loop oligo (217), the second additional oligo (219) and the second probe (201) with ligation.

FIG. 2C illustrates a principle structure of a probe set having a plurality of probe entities according to an embodiment of the invention. The probes correspond to those of FIG. 2A, except that in this embodiment the loop oligo is positioned directly adjacent to the first probe such that a nick is left between 210 and 213.

FIG. 2D illustrates a principle structure of a probe set having a plurality of probe entities according to an embodiment of the invention. The probes correspond to those of FIG. 2A, except that in this embodiment the loop oligo is positioned directly adjacent to the first probe such that a nick is left between 210 and 213 and an additional oligonucleotide (219) is aligned to the bridge oligo such that nicks are left between 215 and 219, and 219 and 207, permitting joining the first probe (202), the loop oligo (217), the additional oligo (219) and the second probe (201) with ligation.

FIG. 2E illustrates a principle structure of a probe set having a plurality of probe entities according to an embodiment of the invention. The probes correspond to those of FIG. 2A, except that in this embodiment the loop oligo is positioned directly adjacent to the second probe such that a nick is left between 215 and 207.

FIG. 2F illustrates a principle structure of a probe set having a plurality of probe entities according to an embodiment of the invention. The probes correspond to those of FIG. 2A, except that in this embodiment the loop oligo is positioned directly adjacent to the second probe such that a nick is left between 215 and 207 and an additional oligonucleotide (219) is aligned to the bridge oligo such that nicks are left between 210 and 219, and 219 and 213, permitting joining the first probe (202), the additional oligo (219) the loop oligo (217), and the second probe (201) with ligation.

FIG. 2G illustrates a principle structure of a probe set having a plurality of probe entities according to an embodiment of the invention. The probes correspond to those of FIG. 2A, except that this embodiment comprises two bridge oligos (200 and 220), wherein bridge oligo 200 comprises 209 and 214 and bridge oligo 220 comprises 208 and 216.

FIG. 2H illustrates a principle structure of a probe set having a plurality of probe entities according to an embodiment of the invention. The probes correspond to those of FIG. 2A, except that the bridge oligo 200 contains sequences (219) that comprise a plurality of universal base analogues to permit the incorporation of random sequences suitable for use as molecular barcode for target enumeration. One or both 219 sequences indicated may be present.

FIG. 2I illustrates a principle structure of a probe quadruplet according to an embodiment herein. The plurality of probe entities includes a first probe (202), a second probe (201), a bridge oligo (200) and a loop oligo (217). Here the probe complex contains gaps between the first probe and the loop oligo (210 and 213), between the second probe and the and the loop oligo (207 and 215) and between the target-specific parts of the first and second probes (203 and 204). These gaps are filled by introducing a polymerase and one or more nucleotides. For this process, a mixture of Stoffel fragment, Taq polymerase or Phusion polymerase, and DNA ligase such as Ampligase can be used. The polymerase fills these gaps and the subsequent action of the DNA ligase results in ligation of the probe, bridge and loop oligos into a circular complex.

15-25 bases of the first probe includes a bridge binding sequence (210), that optionally includes chemically modified bases for efficient bridge oligo binding. The first probe further includes a binding site for an amplification primer (221) and a barcode sequence (222), and 15-30 bases from the 5' end, a sequence binding to the genetic target (203). Some or all of the nucleotides of 210 may include chemical modifications that increase the affinity of the probes to the target or the bridge (209). The last base of the first probe optionally includes a phosphate moiety for enzymatic ligation or a modification that permits chemical ligation to the 5' end of the adjacent probe (205).

The first base of the second probe optionally includes a phosphate moiety for enzymatic ligation or a modification that permits chemical ligation to the 5' end of the adjacent probe (206). The 15-30 bases from the 5' end of the second probe include a part of the second probe that binds to the genetic target (204). The second probe further includes a binding site for an amplification primer (223), a sequencing adapter sequence (224), an optional recognition site for a restriction endonuclease such as EcoRI or a homing endonuclease (225) and another sequencing adapter sequence (207). The last 15-25 bases of the second probe (207), are reverse complementary to the bridge oligo (208). Some or all of the nucleotides of 203, 204, 207 or 210 may include chemical modifications that increase the affinity of the probes to the target or the bridge oligo.

The first 15-25 bases from the 5' end of the bridge oligo (209), are reverse complementary to the bridge-oligo specific sequence of the first probe (210), and optionally include chemically modified nucleotides for increased binding. The last 15-25 bases of the bridge oligo (208), are reverse complementary to the sequence of the second probe (207), and optionally include chemically modified nucleotides for increased binding. The part of the bridge oligo not reverse-complementary with either end of the loop oligo (220) optionally contains a recognition site for a restriction endonuclease to permit enzymatic degradation of non-loop-containing structures. The 5' end of the bridge oligo optionally includes a capture moiety (211) used for capturing the probe complexes. Furthermore, the bridge oligo comprises sequences 214 and 216, complementary to sequences 213 and 215 of the loop oligo. The 3' end of the bridge oligo optionally includes a phosphate (or other cleavable) moiety (212) to prevent extension during gap fill.

The first 15-25 bases from the 5' end of the loop oligo (215) are reverse complementary to bridge oligo sequence 216. The loop oligo comprises a loop region optionally comprising a barcode (218). The last 15-25 bases of the loop oligo (213) are reverse complementary to bridge oligo sequence 214.

## Claims

1. A method for the high-throughput amplification, and optional subsequent detection, of one or more target nucleotide sequence in a plurality of samples, the method comprising the steps of:
(i) providing for each target nucleotide sequence in each of the samples:
a first probe, a second probe, at least one loop oligo and a bridge oligo or a plurality of bridge oligonucleotides capable of annealing to the loop oligo to form a bridge oligo complex,
wherein the first probe comprises a first bridge oligo-specific sequence at the 5' end of the first probe, optionally a first sequence barcode, and a first target specific portion at the 3' end of the first probe;
wherein the second probe comprises a second target specific portion at the 5' end of the second probe, optionally a second sequence barcode,
and a second bridge oligo-specific sequence at the 3' end of the second probe;
wherein the loop oligo comprises, starting from the 5' end of the molecule, a third bridge oligo-specific sequence, a loop sequence and a fourth bridge oligo-specific sequence, wherein the loop sequence optionally comprises a third sequence barcode and wherein the loop sequence contains two sequences that are capable of annealing to each other such that a double-stranded portion is formed, wherein the double-stranded portion comprises a recognition site for a nicking endonuclease; wherein the bridge oligo or plurality of bridge oligonucleotides contains sequences complementary to the first bridge oligo-specific sequence and the second bridge oligo-specific sequence in the first probe and the second probe, respectively, and sequences complementary to the third bridge oligo-specific sequence and the fourth bridge oligo-specific sequence in the loop oligo and wherein the bridge oligo or plurality of bridge oligonucleotides optionally comprises a fourth sequence barcode; wherein at least one of: the first sequence barcode, the second sequence barcode, the third sequence barcode or the fourth sequence barcode is present;
(ii) contacting, for each of the one or more target nucleotide sequence, the first probe and the second probe with, preferably for each of the samples in a separate tube, the bridge oligo or plurality of bridge oligonucleotides and optionally the loop oligo, and allow self-annealing into probe complexes, wherein, if the loop oligo was added, optionally, further oligonucleotides complementary to the bridge oligo or to the plurality of bridge oligonucleotides are included to fill single-stranded gaps, if present, between the loop oligo and the first probe and/or between the loop oligo and the second probe;
(iia) if the loop oligo was added in step (ii), optionally performing polymerase extension to fill single-stranded gaps within the annealed probe complexes, if present;
(iib) if the loop oligo was added in step (ii), optionally ligating the double-stranded portions of the probe complexes,
wherein step (iib) may be performed simultaneously with step (iia);
(iii) contacting nucleic acids present in each of the samples to be tested for the target nucleotide sequences with the probe complexes and the loop oligo, if the loop oligo was not added in step (ii);
(iv) allowing the first target specific portion and the second target specific portion of the respective first probe and the second probe to hybridize to essentially adjacent sections on the target sequence and, if the loop oligo was added in step (iii), allowing the loop oligo to hybridize to the bridge oligo or plurality of bridge oligonucleotides, thereby forming hybridization complexes;
(v) optionally pooling the hybridization complexes from the plurality of samples;
(vi) ligating the probes in the hybridization complexes to provide ligated ligation complexes;
(vii) amplifying nucleic acids from the one or more ligated ligation complexes using rolling circle amplification with a strand-displacing polymerase and allowing annealing of the two sequences in the loop sequence that are capable of annealing to each other, thereby obtaining concatemeric molecules comprising single-stranded portions and loop structures having double-stranded portions comprising the recognition sites for a nicking endonuclease;
(viii) nicking the double-stranded portion with a nicking endonuclease having specificity for said recognition sites;
(ix) performing a denaturation step such that the nicked concatemeric molecules disintegrate into separate segments having complementary ends; and
(x) allowing intramolecular annealing of the complementary ends of the segments and ligating the separate segments intramolecularly, thereby obtaining amplified circular molecular templates;
and, optionally, performing the additional steps of:
(xi) subjecting the amplified circular molecular templates obtained in step (x) to high-throughput sequencing technology to determine the barcode sequence(s); and
(xii) identifying the presence and/or number of the target nucleotide sequence in the plurality of samples by determination of at least part of the first target specific portion and/or the second target specific portion, and/or at least part of the first barcode and/or the second barcode, and/or at least part of the third barcode and/or at least part of the fourth barcode,
wherein steps (v) and (vi) may be performed in any order.

2. Method according to claim 1, wherein the method comprises steps (xi) and (xii).

3. Method according to claim 1 or 2, wherein the two sequences that are capable of annealing to each other contained within the loop sequence each have a length of at least 5 bases, such as at least 6, 7, 8, 9 or at least 10 bases, for example a length of between 10 and 50 bases, such as between 10 and 25 bases or between 10 and 15 bases.

4. Method according to any one of the preceding claims, wherein the nicking endonuclease is Nb.BbvCI or Nb.BsrDI.

5. Method according to any one of the preceding claims, wherein the bridge oligo, or one or more oligonucleotides of the plurality of bridge oligonucleotides, comprises, in a region not complementary to the first probe, the second probe or the loop oligo, a plurality of universal base analogues to permit the incorporation of random sequences suitable for use as molecular barcode for target enumeration, and wherein, as part of step (vi), a gap filling step is performed using polymerase and nucleotides in order to generate such random sequences.

6. Method according to claim 5, wherein said plurality of universal base analogues is a plurality of 5-nitroindoles.

7. Method according to any one of the preceding claims, wherein subsequent to step (v), but prior to step (vii), a step (a) and a step (b) are performed, wherein step (a) comprises allowing the ligated ligation complexes to dissociate from the target nucleotide sequence and step (b) comprises adding a target-specific probe comprising a sequence corresponding to the target nucleotide sequence, wherein said target-specific probe is capable of annealing with the ligated ligation complexes, and allowing the target-specific probe to anneal to the ligated ligation complexes thereby forming amplification templates, and
wherein, in step (vii), said amplification templates are amplified by rolling circle amplification with a strand-displacing polymerase.

8. Method according to any one of the preceding claims, wherein at least one of: the first probe, the second probe, the loop oligo, the bridge oligo, or an oligonucleotide of the plurality of bridge oligonucleotides, comprises a first capture moiety, and wherein between steps (iv) and (v) an intermediate step (iv)(a) is performed which comprises bringing the hybridization complex in contact with a solid support comprising a second capture moiety, allowing the first capture moiety and the second capture moiety to interact such that the hybridization complexes become linked to the solid support and separating the solid-support-linked hybridization complexes from components of the samples that are not linked to the solid-support.

9. Method according to any one of the preceding claims, wherein the plurality of samples includes a blood sample, a saliva sample, a urine sample or a feces sample.

10. Method according to any one of the preceding claims, wherein the bridge oligo or an oligonucleotide of the plurality of bridge oligonucleotides comprises:
(i) one to five 3' protruding bases, and/or
(ii) 3' phosphate, and/or
(iii) one or more phosphorothioate modifications within three positions from the 3' end, and/or
(iv) another modification that protects the bridge from exonuclease activity and/or prevent amplification from the 3' end.

11. Method according to any one of the preceding claims, wherein one or more of: the first probe, the second probe, the loop oligo, the bridge oligo or an oligonucleotide of the plurality of bridge oligonucleotides are modified to permit chemical ligation.

12. Method according to any one of the preceding claims, wherein the bridging portion of the first probe or the second probe, or both, or the loop oligo, the bridge oligo or an oligonucleotide of the plurality of bridge oligonucleotides comprise(s) chemically modified bases to permit improved binding to the bridge oligo or bridge oligo complex.

13. Method according to any one of the preceding claims, wherein the first target specific portion, the second target specific portion, the first bridge oligo-specific sequences, and/or the second bridge oligo-specific sequences, contain independently from one another, one or more chemically modified nucleotide.

14. Method according to any one of the preceding claims, wherein the bridge oligo, or an oligonucleotide of the plurality of bridge oligonucleotides, comprises one or more chemically modified nucleotides.

15. Method according to any one of the preceding claims, wherein step (vii) is performed using a phi29 polymerase or a Bst polymerase.

16. Method according to any one of the preceding claims, wherein genetic target enumeration is permitted by counting the number of molecular barcodes per target and per sample.

17. Method according to any one of the preceding claims, wherein for two or more samples or for two or more locus/allele combinations, barcode sequences are used to genotype the sample(s) for one or more sequences and/or polymorphisms, such as SNPs and/or indels.

## Patentansprüche

1. Verfahren für die Hochdurchsatzamplifikation und optional anschließende Erfassung einer oder mehrerer Zielnucleotidsequenzen in einer Vielzahl von Proben, das Verfahren umfassend die Schritte:
(i) Bereitstellen für jede Zielnucleotidsequenz in jeder der Proben: einer ersten Sonde, einer zweiten Sonde, mindestens eines Loop-Oligos und eines Brückenoligos oder einer Vielzahl von Brückenoligonucleotiden, die in der Lage sind, an das Loop-Oligo zu tempern, um einen Brückenoligokomplex auszubilden,
wobei die erste Sonde eine erste brückenoligospezifische Sequenz an dem 5'-Ende der ersten Sonde, optional einen ersten Sequenzbarcode, und einen ersten zielspezifischen Abschnitt an dem 3'-Ende der ersten Sonde umfasst;
wobei die zweite Sonde einen zweiten zielspezifischen Abschnitt an dem 5'-Ende der zweiten Sonde, optional einen zweiten Sequenzbarcode, und eine zweite brückenoligospezifische Sequenz an dem 3'-Ende der zweiten Sonde umfasst;
wobei das Loop-Oligo, beginnend von dem 5'-Ende des Moleküls, eine dritte brückenoligospezifische Sequenz, eine Loop-Sequenz und eine vierte brückenoligospezifische Sequenz umfasst, wobei die Loop-Sequenz optional einen dritten Sequenzbarcode umfasst und wobei die Loop-Sequenz zwei Sequenzen enthält, die in der Lage sind, aneinander derart zu tempern, dass ein doppelsträngiger Abschnitt ausgebildet wird, wobei der doppelsträngige Abschnitt eine Erkennungsstelle für eine Strangbruch-Endonuclease umfasst; wobei das Brückenoligo oder die Vielzahl von Brückenoligonucleotiden Sequenzen, die komplementär zu der ersten brückenoligospezifischen Sequenz und der zweiten brückenoligospezifischen Sequenz in der ersten Sonde beziehungsweise der zweiten Sonde sind, und Sequenzen enthält, die komplementär zu der dritten brückenoligospezifischen Sequenz und der vierten brückenoligospezifischen Sequenz in dem Barcode-Loop-Oligo sind und wobei das Brückenoligo oder die Vielzahl von Brückenoligonucleotiden optional einen vierten Sequenzbarcode umfasst;
wobei mindestens einer von: dem ersten Sequenzbarcode, dem zweiten Sequenzbarcode, dem dritten Sequenzbarcode oder dem vierten Sequenzbarcode vorhanden ist;
(ii) Kontaktieren, für jede der einen oder der mehreren Zielnucleotidsequenzen, der ersten Sonde und der zweiten Sonde mit, vorzugsweise für jede der Proben in einem separaten Röhrchen, dem Brückenoligo oder der Vielzahl von Brückenoligonucleotiden und optional dem Loop-Oligo, und Ermöglichen eines Selbsttemperns zu Sondenkomplexen, wobei, falls das Loop-Oligo zugegeben wurde, optional weitere Oligonucleotide komplementär zu dem Brückenoligo oder zu der Vielzahl von Brückenoligonucleotiden eingeschlossen sind, um einzelsträngige Lücken, falls vorhanden, zwischen dem Loop-Oligo und der ersten Sonde und/oder zwischen dem Loop-Oligo und der zweiten Sonde zu füllen;
(iia) falls das Loop-Oligo in Schritt (ii) zugegeben wurde, optional Durchführen einer Polymeraseverlängerung, um einzelsträngige Lücken innerhalb der getemperten Sondenkomplexe, falls vorhanden, zu füllen;
(iib) falls das Loop-Oligo in Schritt (ii) zugegeben wurde, optional Ligieren der doppelsträngigen Abschnitte der Sondenkomplexe,
wobei Schritt (iib) gleichzeitig mit Schritt (iia) durchgeführt werden kann;
(iii) Kontaktieren von Nucleinsäuren, die in jeder der Proben vorhanden ist, die auf die Zielnucleotidsequenzen getestet werden sollen, mit den Sondenkomplexen und dem Loop-Oligo, falls das Loop-Oligo nicht in Schritt (ii) zugegeben wurde;
(iv) Ermöglichen dem ersten zielspezifischen Abschnitt und dem zweiten zielspezifischen Abschnitt der jeweiligen ersten Sonde und der zweiten Sonde, an im Wesentlichen benachbarte Bereiche auf der Zielsequenz zu hybridisieren und, falls das Loop-Oligo in Schritt (iii) zugegeben wurde, Ermöglichen dem Loop-Oligo, an das Brückenoligo oder die Vielzahl von Brückenoligonucleotiden zu hybridisieren, wobei dadurch Hybridisierungskomplexe ausgebildet werden;
(v) optional Zusammenführen der Hybridisierungskomplexe aus der Vielzahl von Proben;
(vi) Ligieren der Sonden in den Hybridisierungskomplexen, um ligierte Ligationskomplexe bereitzustellen;
(vii) Amplifizieren von Nucleinsäuren aus dem einen oder den mehreren ligierten Ligationskomplexen unter Verwendung einer Walzkreisamplifikation mit einer strangverdrängenden Polymerase und Ermöglichen des Temperns der zwei Sequenzen in der Loop-Sequenz, die in der Lage sind, aneinander zu tempern, wobei dadurch konkatemere Moleküle erhalten werden, umfassend einzelsträngige Abschnitte und Loop-Strukturen, die doppelsträngige Abschnitte aufweisen, umfassend die Erkennungsstellen für eine Strangbruch-Endonuclease;
(viii) Strangbrechen des doppelsträngigen Abschnitts mit einer Strangbruch-Endonuclease, die eine Spezifität für die Erkennungsstellen aufweist;
(ix) Durchführen eines Denaturierungsschritts derart, dass die stranggebrochenen konkatemeren Moleküle in separate Segmente zerfallen, die komplementäre Enden aufweisen; und
(x) Ermöglichen des intramolekularen Temperns der komplementären Enden der Segmente und intramolekulares Ligieren der separaten Segmente, wobei dadurch amplifizierte kreisförmige molekulare Vorlagen erhalten werden;
und, optional, Durchführen der zusätzlichen Schritte:
(xi) Unterziehen der amplifizierten kreisförmigen molekularen Vorlagen, die in Schritt (x) erhalten werden, einer Hochdurchsatz-Sequenzierungstechnologie, um die Barcodesequenz(en) zu bestimmen; und
(xiii) Identifizieren des Vorhandenseins und/oder Anzahl der Zielnucleotidsequenz in der Vielzahl von Proben durch Bestimmung mindestens eines Teils des ersten zielspezifischen Abschnitts und/oder des zweiten zielspezifischen Abschnitts, und/oder mindestens eines Teils des ersten Barcodes und/oder des zweiten Barcodes, und/oder mindestens eines Teils des dritten Barcodes und/oder mindestens eines Teils des vierten Barcodes,
wobei Schritte (v) und (vi) in beliebiger Reihenfolge durchgeführt werden können.

2. Verfahren nach Anspruch 1, wobei das Verfahren Schritte (xi) und (xii) umfasst.

3. Verfahren nach Anspruch 1 oder 2, wobei die zwei Sequenzen, die in der Lage sind, aneinander zu tempern, die innerhalb der Loop-Sequenz enthalten sind, jeweils eine Länge von mindestens 5 Basen, wie mindestens 6, 7, 8, 9 oder mindestens 10 Basen, zum Beispiel eine Länge zwischen 10 und 50 Basen, wie zwischen 10 und 25 Basen oder zwischen 10 und 15 Basen, aufweisen.

4. Verfahren nach einem der vorstehenden Ansprüche, wobei die Strangbruch-Endonuclease Nb.BbvCI oder Nb.BsrDI ist.

5. Verfahren nach einem der vorstehenden Ansprüche, wobei das Brückenoligo oder ein oder mehrere Oligonucleotide der Vielzahl von Brückenoligonucleotiden in einer Region, die nicht komplementär zu der ersten Sonde, der zweiten Sonde oder dem Loop-Oligo ist, eine Vielzahl von universellen Basenanaloga umfasst, um die Einbindung von Zufallssequenzen zu gestatten, die zur Verwendung als molekularer Barcode für eine Zielzählung geeignet sind, und wobei, als Teil von Schritt (vi), ein Schritt zum Füllen von Lücken unter Verwendung von Polymerase und Nucleotiden durchgeführt wird, um derartige Zufallssequenzen zu generieren.

6. Verfahren nach Anspruch 5, wobei die Vielzahl von universellen Basenanaloga eine Vielzahl von 5-Nitroindolen ist.

7. Verfahren nach einem der vorstehenden Ansprüche, wobei anschließend an Schritt (v), aber vor Schritt (vii), ein Schritt (a) und ein Schritt (b) durchgeführt werden, wobei Schritt (a) das Ermöglichen umfasst, dass sich die ligierten Ligationskomplexe von der Zielnucleotidsequenz loslösen, und Schritt (b) ein Hinzufügen einer zielspezifischen Sonde umfasst, umfassend eine Sequenz, die der Zielnucleotidsequenz entspricht, wobei die zielspezifische Sonde in der Lage ist, mit den ligierten Ligationskomplexen zu tempern, und ermöglicht, dass die zielspezifische Sonde an die ligierten Ligationskomplexe tempert, wodurch Amplifikationsvorlagen ausgebildet werden, und
wobei in Schritt (vii) die Amplifikationsvorlagen durch Walzkreisamplifikation mit einer strangverdrängenden Polymerase amplifiziert werden.

8. Verfahren nach einem der vorstehenden Ansprüche, wobei mindestens eines von: der ersten Sonde, der zweiten Sonde, dem Loop-Oligo, dem Brückenoligo oder einem Oligonucleotid der Vielzahl von Brückenoligonucleotiden eine erste Erfassungseinheit umfasst, und wobei zwischen Schritten (iv) und (v) ein Zwischenschritt (iv)(a) durchgeführt wird, der ein Inkontaktbringen des Hybridisierungskomplexes mit einem festen Träger, umfassend eine zweite Erfassungseinheit, das Ermöglichen der ersten Erfassungseinheit und der zweiten Erfassungseinheit, derart zusammenwirken, dass die Hybridisierungskomplexe mit dem festen Träger verbunden werden, und das Trennen der mit dem festen Träger verbundenen Hybridisierungskomplexe von Komponenten der Proben umfasst, die nicht mit dem festen Träger verbunden sind.

9. Verfahren nach einem der vorstehenden Ansprüche, wobei die Vielzahl von Proben eine Blutprobe, eine Speichelprobe, eine Urinprobe oder eine Stuhlprobe einschließt.

10. Verfahren nach einem der vorstehenden Ansprüche, wobei das Brückenoligo oder ein Oligonucleotid der Vielzahl von Brückenoligonucleotiden umfasst:
(i) eine bis fünf 3' vorstehende Basen und/oder
(ii) 3'-Phosphat, und/oder
(iii) eine oder mehrere Phosphorothioatmodifikationen innerhalb von drei Positionen von dem 3'-Ende, und/oder
(iv) eine weitere Modifikation, die die Brücke vor Exonucleaseaktivität schützt und/oder eine Amplifikation von dem 3'-Ende verhindert.

11. Verfahren nach einem der vorstehenden Ansprüche, wobei eines oder mehrere von: der ersten Sonde, der zweiten Sonde, dem Loop-Oligo, dem Brückenoligo oder einem Oligonucleotid der Vielzahl von Brückenoligonucleotiden modifziert werden, um eine chemische Ligation zu gestatten.

12. Verfahren nach einem der vorstehenden Ansprüche, wobei der Brückenabschnitt der ersten Sonde oder der zweiten Sonde oder beider oder das Loop-Oligo, das Brückenoligo oder ein Oligonucleotid der Vielzahl von Brückenoligonucleotiden chemisch modifizierte Basen umfasst/umfassen, um eine verbesserte Bindung an das Brückenoligo oder den Brückenoligokomplex zu gestatten.

13. Verfahren nach einem der vorstehenden Ansprüche, wobei der erste zielspezifische Abschnitt, der zweite zielspezifische Abschnitt, die ersten brückenoligospezifischen Sequenzen und/oder die zweiten brückenoligospezifischen Sequenzen unabhängig voneinander ein oder mehrere chemisch modifizierte Nucleotide enthalten.

14. Verfahren nach einem der vorstehenden Ansprüche, wobei das Brückenoligo oder ein Oligonucleotid der Vielzahl von Brückenoligonucleotiden ein oder mehrere chemisch modifizierte Nucleotide umfasst.

15. Verfahren nach einem der vorstehenden Ansprüche, wobei Schritt (vii) unter Verwendung einer phi29-Polymerase oder einer Bst-Polymerase durchgeführt wird.

16. Verfahren nach einem der vorstehenden Ansprüche , wobei die genetische Zielauszählung durch Zählen der Anzahl von molekularen Barcodes pro Ziel und pro Probe gestattet wird.

17. Verfahren nach einem der vorstehenden Ansprüche, wobei für zwei oder mehr Proben oder für zwei oder mehr Locus-/Allelkombinationen Barcode-Sequenzen verwendet werden, um die Probe(n) für eine oder mehrere Sequenzen und/oder Polymorphismen, wie SNPs und/oder Indels, zu genotypisieren.

## Revendications

1. Procédé destiné à l'amplification à haut rendement, et à la détection ultérieure facultative, d'une ou plusieurs séquences nucléotidiques cibles dans une pluralité d'échantillons, le procédé comprenant les étapes consistant à :
(i) fournir pour chaque séquence nucléotidique cible dans chacun des échantillons : une première sonde, une seconde sonde, au moins une oligo-boucle et un oligo-pont ou une pluralité d'oligonucléotides de pont capable de renaturation à l'oligo-boucle pour former un complexe d'oligo-pont,
dans lequel la première sonde comprend une première séquence spécifique d'oligo-pont au niveau de l'extrémité 5' de la première sonde, facultativement un premier code-barres de séquence, et une première partie spécifique de cible au niveau de l'extrémité 3' de la première sonde ;
dans lequel la seconde sonde comprend une seconde partie spécifique de cible au niveau de l'extrémité 5' de la seconde sonde, facultativement un deuxième code-barres de séquence, et une deuxième séquence spécifique d'oligo-pont au niveau de l'extrémité 3' de la seconde sonde ;
dans lequel l'oligo-boucle comprend, en partant de l'extrémité 5' de la molécule, une troisième séquence spécifique d'oligo-pont, une séquence de boucle et une quatrième séquence spécifique d'oligo-pont, dans lequel la séquence de boucle comprend facultativement un troisième code-barres de séquence et dans lequel la séquence de boucle contient deux séquences qui sont capables de renaturation l'une à l'autre de telle sorte qu'une partie à double brin est formée, dans lequel la partie à double brin comprend un site de reconnaissance pour une endonucléase de coupure ;
dans lequel l'oligo-pont ou la pluralité d'oligonucléotides de pont contient des séquences complémentaires à la première séquence spécifique d'oligo-pont et à la deuxième séquence spécifique d'oligo-pont dans la première sonde et la seconde sonde, respectivement, et des séquences complémentaires à la troisième séquence spécifique d'oligo-pont et à la quatrième séquence spécifique d'oligo-pont dans l'oligo-boucle et dans lequel l'oligo-pont ou la pluralité d'oligonucléotides de pont comprend facultativement un quatrième code-barres de séquence ;
dans lequel au moins l'un parmi : le premier code-barres de séquence, le deuxième code-barres de séquence, le troisième code-barres de séquence ou le quatrième code-barres de séquence est présent ;
(ii) mettre en contact, pour chacune parmi la ou les séquences nucléotidiques cibles, la première sonde et la seconde sonde avec, de préférence pour chacun des échantillons dans un tube indépendant, l'oligo-pont ou la pluralité d'oligonucléotides de pont et facultativement l'oligo-boucle, et permettre une auto-renaturation en complexes de sonde, dans lequel, si l'oligo-boucle a été ajoutée, facultativement, d'autres oligonucléotides complémentaires à l'oligo-pont ou à la pluralité d'oligonucléotides de pont sont inclus pour remplir des espaces à simple brin, s'ils sont présents, entre l'oligo-boucle et la première sonde et/ou entre l'oligo-boucle et la seconde sonde ;
(iia) si l'oligo-boucle a été ajoutée à l'étape (ii), mettre en œuvre facultativement une extension par polymérase pour remplir des espaces à simple brin au sein des complexes de sonde renaturés, s'ils sont présents ;
(iib) si l'oligo-boucle a été ajoutée à l'étape (ii), ligaturer facultativement les parties à double brin des complexes de sonde,
dans lequel l'étape (iib) peut être mise en œuvre simultanément à l'étape (iia) ;
(iii) mettre en contact des acides nucléiques présents dans chacun des échantillons à tester pour les séquences nucléotidiques cibles avec les complexes de sonde et l'oligo-boucle, si l'oligo-boucle n'a pas été ajoutée à l'étape (ii) ;
(iv) laisser la première partie spécifique de cible et la seconde partie spécifique de cible de la première sonde et de la seconde sonde respective s'hybrider à des sections sensiblement adjacentes sur la séquence cible et, si l'oligo-boucle a été ajoutée à l'étape (iii), laisser l'oligo-boucle s'hybrider à l'oligo-pont ou à la pluralité d'oligonucléotides de pont, en formant de ce fait des complexes d'hybridation ;
(v) facultativement mettre en commun les complexes d'hybridation provenant de la pluralité d'échantillons ;
(vi) ligaturer les sondes dans les complexes d'hybridation pour fournir des complexes de ligature ligaturés ;
(vii) amplifier des acides nucléiques provenant du ou des complexes de ligature ligaturés à l'aide d'une amplification par cercle roulant avec une polymérase de déplacement de brin et permettre une renaturation des deux séquences dans la séquence de boucle qui sont capables de renaturation l'une à l'autre, en obtenant de ce fait des molécules concatémères comprenant des parties à simple brin et des structures de boucle ayant des parties à double brin comprenant les sites de reconnaissance pour une endonucléase de coupure ;
(viii) couper la partie à double brin avec une endonucléase de coupure ayant une spécificité pour lesdits sites de reconnaissance ;
(ix) mettre en œuvre une étape de dénaturation de telle sorte que les molécules concatémères coupées se désintègrent en segments indépendants ayant des extrémités complémentaires ; et
(x) permettre une renaturation intramoléculaire des extrémités complémentaires des segments et ligaturer les segments indépendants de façon intramoléculaire, en obtenant de ce fait des modèles moléculaires circulaires amplifiés ;
et, facultativement, mettre en œuvre les étapes supplémentaires consistant à :
(xi) soumettre les modèles moléculaires circulaires amplifiés obtenus à l'étape (x) à une technologie de séquençage à haut rendement pour déterminer la ou les séquence(s) de code-barres ; et
(xii) identifier la présence et/ou le nombre des séquences nucléotidiques cibles dans la pluralité d'échantillons par détermination d'au moins une partie de la première partie spécifique de cible et/ou de la deuxième partie spécifique de cible, et/ou d'au moins une partie du premier code-barres et/ou du deuxième code-barres, et/ou d'au moins une partie du troisième code-barres et/ou d'au moins une partie du quatrième code-barres,
dans lequel les étapes (v) et (vi) peuvent être mises en œuvre dans n'importe quel ordre.

2. Procédé selon la revendication 1, dans lequel le procédé comprend les étapes (xi) et (xii).

3. Procédé selon la revendication 1 ou 2, dans lequel les deux séquences qui sont capables de renaturation l'une à l'autre contenues à l'intérieur de la séquence de boucle ont chacune une longueur d'au moins 5 bases, telle qu'au moins 6, 7, 8, 9 ou au moins 10 bases, par exemple une longueur comprise entre 10 et 50 bases, telle qu'entre 10 et 25 bases ou entre 10 et 15 bases.

4. Procédé selon l'une quelconque des revendications précédentes, dans lequel l'endonucléase de coupure est Nb.BbvCI ou Nb.BsrDI.

5. Procédé selon l'une quelconque des revendications précédentes, dans lequel l'oligo-pont, ou un ou plusieurs oligonucléotides de la pluralité d'oligonucléotides de pont, comprennent, dans une région non complémentaire à la première sonde, à la seconde sonde ou à l'oligo-boucle, une pluralité d'analogues de bases universelles pour permettre l'incorporation de séquences aléatoires appropriées pour une utilisation en tant que code-barres moléculaire pour énumération de cibles, et dans lequel, dans le cadre de l'étape (vi), une étape de remplissage d'espace est effectuée à l'aide de polymérase et de nucléotides afin de générer de telles séquences aléatoires.

6. Procédé selon la revendication 5, dans lequel ladite pluralité d'analogues de bases universelles est une pluralité de 5-nitroindoles.

7. Procédé selon l'une quelconque des revendications précédentes, dans lequel, après l'étape (v), mais avant l'étape (vii), une étape (a) et une étape (b) sont mises en œuvre, dans lequel l'étape (a) comprend le fait de permettre aux complexes de ligature ligaturés de se dissocier de la séquence nucléotidique cible et l'étape (b) comprend l'ajout d'une sonde spécifique à la cible comprenant une séquence correspondant à la séquence nucléotidique cible, dans lequel ladite sonde spécifique à la cible est capable de s'aligner avec les complexes de ligature ligaturés et de permettre à la sonde spécifique à la cible de s'aligner avec les complexes de ligature ligaturés, en formant de ce fait des gabarits d'amplification, et
dans lequel, à l'étape (vii), lesdits modèles d'amplification sont amplifiés par amplification en cercle roulant à l'aide d'une polymérase à déplacement de brin.

8. Procédé selon l'une quelconque des revendications précédentes, dans lequel au moins l'un parmi la première sonde, la seconde sonde, l'oligo-boucle, l'oligo-pont, ou un oligonucléotide de la pluralité d'oligonucléotides de pont, comprend un premier fragment de capture, et dans lequel, entre les étapes (iv) et (v), une étape intermédiaire (iv)(a) est mise en œuvre, qui comprend la mise en contact du complexe d'hybridation avec un support solide comprenant un second fragment de capture, le fait de permettre au premier fragment de capture et au second fragment de capture d'interagir de telle sorte que les complexes d'hybridation deviennent liés au support solide et la séparation des complexes d'hybridation liés au support solide par rapport à des composants des échantillons qui ne sont pas liés au support solide.

9. Procédé selon l'une quelconque des revendications précédentes, dans lequel la pluralité d'échantillons comporte un échantillon de sang, un échantillon de salive, un échantillon d'urine ou un échantillon de selles.

10. Procédé selon l'une quelconque des revendications précédentes, dans lequel l'oligo-pont ou un oligonucléotide de la pluralité d'oligonucléotides de pont comprend :
(i) une à cinq bases saillantes 3', et/ou
(ii) du phosphate 3', et/ou
(iii) une ou plusieurs modifications phosphorothioate au sein de trois positions à partir de l'extrémité 3', et/ou
(iv) une autre modification qui protège le pont d'une activité d'exonucléase et/ou empêche une amplification à partir de l'extrémité 3'.

11. Procédé selon l'une quelconque des revendications précédentes, dans lequel un ou plusieurs parmi : la première sonde, la seconde sonde, l'oligo-boucle, l'oligo-pont ou un oligonucléotide de la pluralité d'oligonucléotides de pont sont modifiés pour permettre une ligature chimique.

12. Procédé selon l'une quelconque des revendications précédentes, dans lequel la partie de pontage de la première sonde ou de la seconde sonde, ou l'une et l'autre, ou l'oligo-boucle, l'oligo-pont ou un oligonucléotide de la pluralité d'oligonucléotides de pont comprend/comprennent des bases modifiées chimiquement pour permettre une liaison améliorée à l'oligo-pont ou au complexe d'oligo-pont.

13. Procédé selon l'une quelconque des revendications précédentes, dans lequel la première partie spécifique de cible, la seconde partie spécifique de cible, les premières séquences spécifiques d'oligo-pont, et/ou les secondes séquences spécifiques d'oligo-pont, contiennent indépendamment les unes des autres, un ou plusieurs nucléotides chimiquement modifiés.

14. Procédé selon l'une quelconque des revendications précédentes, dans lequel l'oligo-pont, ou un oligonucléotide de la pluralité d'oligonucléotides de pont, comprend un ou plusieurs nucléotides chimiquement modifiés.

15. Procédé selon l'une quelconque des revendications précédentes, dans lequel l'étape (viii) est mise en œuvre à l'aide d'une polymérase phi29 ou d'une polymérase Bst.

16. Procédé selon l'une quelconque des revendications précédentes, dans lequel un dénombrement de cibles génétiques est permis en comptant le nombre de code-barres moléculaires par cible et par échantillon.

17. Procédé selon l'une quelconque des revendications précédentes, dans lequel pour deux échantillons ou plus ou pour deux combinaisons de locus/allèle ou plus, des séquences de code-barres sont utilisées pour génotyper le(s) échantillon(s) pour une ou plusieurs séquences et/ou polymorphismes, tels que des SNP et/ou des indels.
